(19) Europäisches Patentamt / European Patent Office / Office européen des brevets

(11) **EP 4 212 542 A1**

(12) **EUROPEAN PATENT APPLICATION**
published in accordance with Art. 153(4) EPC

(43) Date of publication:
**19.07.2023 Bulletin 2023/29**

(21) Application number: **21865532.2**

(22) Date of filing: **20.04.2021**

(51) International Patent Classification (IPC):
*C07K 7/06* (2006.01)    *C07K 14/61* (2006.01)
*A61K 38/02* (2006.01)    *A61P 19/00* (2006.01)

(52) Cooperative Patent Classification (CPC):
**A61K 38/02; A61P 19/00; C07K 7/06; C07K 14/61**

(86) International application number:
**PCT/CN2021/088266**

(87) International publication number:
**WO 2022/052471 (17.03.2022 Gazette 2022/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.09.2020   CN 202010946404**

(71) Applicant: **Shaanxi Micot Technology Limited
Company**
**Xi'an, Shaanxi 710077 (CN)**

(72) Inventor: **WANG, Cuiqin
Shaanxi 710077 (CN)**

(74) Representative: **ABG Intellectual Property Law,
S.L.**
**Avenida de Burgos, 16D**
**Edificio Euromor**
**28036 Madrid (ES)**

Remarks:
The complete document including Reference
Table(s) and the Sequence Listing(s) can be
downloaded from the EPO website

(54) **BISPECIFIC FUSION POLYPEPTIDE COMPOUND**

(57)     Provided is a polypeptide compound which is X-ID-Y or Y-ID-X, wherein X is a calcium-sensing receptor agonist; ID is a disulfide bond or linker arm within molecules and links X and Y; and Y is a bone growth peptide receptor agonist or a bone marrow mesenchymal stem cell stimulator. Also provided are a pharmaceutical composition comprising the polypeptide compound and a use of the compound and pharmaceutical composition.

**Description**

This application claims the priority of Chinese Patent Application No. 202010946404.5, filed with the China National Intellectual Property Administration on September 10, 2020, and titled "BISPECIFIC FUSION POLYPEPTIDE COMPOUND".

**FIELD**

The present disclosure belongs to the technical field of medicine, and particularly relates to a bispecific fusion polypeptide compound used for treating diseases related to elevated parathyroid hormone and/or blood system-related diseases, and a pharmaceutical composition comprising the compound.

**BACKGROUND**

Chronic kidney disease (CKD) has an incidence of 10.8% in China, of which the proportion of patients with end-stage renal failure and uremia is between 1% and 2%, about 1.38 million people. Symptoms of renal failure include anemia, acidosis, and potassium, sodium, phosphorus and calcium metabolism disorders, etc. The maintenance dialysis treatment of patients with end-stage renal failure and uremia will cause secondary diseases such as diseases related to elevated parathyroid hormone. Among them, secondary hyperparathyroidism (SHPT) is a common long-term complication.

Secondary hyperparathyroidism refers to a chronic compensatory clinical manifestation that the parathyroid glands secrete excessive PTH to increase blood calcium, blood magnesium and reduce blood phosphorus under the long-term stimulation of hypocalcemia, hypomagnesemia or hyperphosphatemia resulting from chronic renal insufficiency, intestinal malabsorption syndrome, Fanconi syndrome, renal tubular acidosis, and vitamin D deficiency or resistance. Progression of SHPT may lead to bone disease, soft tissue calcification, and vascular calcification, affecting patient morbidity and mortality.

Traditional SHPT treatment strategies include surgery, intervention, and drug therapy, among which drug therapy is mainly the use of active vitamin D, traditional phosphorus binders and cinacalcet. Active vitamin D, also known as vitamin D receptor agonist, is currently the most widely used drug for the treatment of SHPT, which reduces the synthesis of PTH by acting on VDR to achieve a therapeutic effect. It is the most common drug at present, but it has the risk of causing hypercalcemia and hyperphosphatemia, which will exacerbate soft tissue calcification and vascular calcification.

The discovery of the function of calcium-sensing receptor (CaSR) has laid the foundation for the discovery of calcium-sensing receptor agonists. Studies have revealed that CaSR is a G-protein coupled receptor expressed in several cell types. CaSR can detect small fluctuations in extracellular calcium ion ($Ca^{2+}$) concentration and response by altering PTH secretion. CaSR inhibits PTH secretion by inhibiting cytosolic transport via $Ca^{2+}$ activation within seconds to minutes, and this process is regulated by protein kinase C (PKC) phosphorylation of the receptor. CaSR is also expressed in osteoblasts and in the kidney, where it regulates renal $Ca^{2+}$ excretion. In 1996, cacimimetics, which is a drug mimicking the calcium structure, was successfully developed. Cacimimetics can act on the CaR transmembrane domain, increase the calcium affinity, shift the calcium dose-response curve to the left, "trick" parathyroid cells to produce a series of signal responses similar to high ionized calcium, activate CaSR, inhibit the synthesis and secretion of PTH, and finally lead to a decrease of blood calcium and blood phosphorus.

Calcium-sensing receptor agonists (calcimimetics) can mimic or even enhance the effect of extracellular calcium ions on parathyroid cells. Calcium-sensing receptor agonists (such as cinacalcet) are used to treat SHPT and give good results. Cinacalcet can allosterically activate CaSR. It has also been found to have a direct inhibitory effect on long-term cultured human parathyroid cells, which can regulate the proliferation cycle of parathyroid cells, thereby affecting the volume of parathyroid glands. Therefore, cinacalcet is also known as "reversible chemical parathyroidectomy" and has become a hot spot of clinical research in recent years. It is now believed that cinacalcet can not only effectively reduce PTH, but also reduce the volume of hyperplastic parathyroid glands to achieve the purpose of parathyroidectomy. In countries like Japan, the proportion of parathyroidectomy has been significantly reduced due to the listing of this drug. Cinacalcet is generally well tolerated, and two most common adverse reactions are hypocalcemia and gastrointestinal side effects. In addition, calcium-sensing receptor agonists in the treatment of hyperparathyroidism mainly work by reducing the level of parathyroid hormone, and have no improvement on other complications of chronic renal failure, such as anemia.

In view of the above, the treatment of SHPT has made great progress, but it is still unable to completely and continuously heal the mineral-bone metabolism disorder. At least half of the patients with SHPT have not been properly treated. In addition, current SHPT drugs have no improvement on the symptoms of anemia associated with renal failure. The treatment of SHPT has not achieved the ideal goal and needs to be further explored. The continued development of compounds that can lower parathyroid hormone levels with better efficacy and fewer side effects is of great significance for the treatment of secondary hyperparathyroidism and renal failure.

**SUMMARY**

In one aspect, a purpose of the present disclosure is to provide an active bispecific fusion polypeptide compound. The active polypeptide compounds provided by the present disclosure have dual-target activities and can simultaneously exert regulatory or therapeutic effects in two different aspects. The active polypeptide compounds disclosed in the present disclosure can not only bind to calcium-sensing receptors and excite the calcium-sensing receptors to reduce the level of parathyroid hormone, but also produce osteogenic growth peptide-like effects.

To achieve the above-mentioned purpose, the present disclosure adopts the following technical solutions.

The present disclosure first provides an active bispecific fusion polypeptide compound having a structure represented by the following formula (Ia) or formula (Ib), or a pharmaceutically acceptable salt thereof:

Y-ID-X formula (Ia),

or

X-ID-Y formula (Ib);

wherein: Y is a calcium-sensing receptor agonist,

ID is an intramolecular disulfide bond or a linker connecting X and Y, and

X is an osteogenic growth peptide-like peptide or a stimulator of bone marrow mesenchymal stem cells.

In some embodiments, Y in the bispecific fusion polypeptide compound of the present disclosure is a peptide chain having an amino acid sequence set forth in formula (IIa) or formula (IIb):

$Xaa_7$-$Xaa_8$-$Xaa_9$-$Xaa_{10}$-$Xaa_{11}$-$Xaa_{12}$-$Xaa_{13}$  formula (IIa),

$Xaa_{13}$-$Xaa_{12}$-$Xaa_{11}$-$Xaa_{10}$-$Xaa_9$-$Xaa_8$-$Xaa_7$  formula (IIb);

wherein, $Xaa_7$ is selected from the group consisting of cysteine, homocysteine and S-methylcysteine;

$Xaa_8$ is selected from the group consisting of alanine and arginine;

$Xaa_9$ is selected from the group consisting of arginine, lysine and histidine;

$Xaa_{10}$ is selected from the group consisting of arginine, alanine, lysine and histidine;

$Xaa_{11}$ is selected from the group consisting of arginine, lysine and histidine;

$Xaa_{12}$ is alanine;

$Xaa_{13}$ is selected from the group consisting of arginine, lysine and histidine; and

the amino terminal of the peptide chain Y is free or chemically modified, and the carboxyl terminal of the peptide chain Y is free or chemically modified.

In the embodiment of the present disclosure, $Xaa_9$, $Xaa_{10}$, $Xaa_{11}$ and $Xaa_{13}$ in the peptide chain Y in the bispecific fusion polypeptide compound of the present disclosure can also be independently selected from the group consisting of 2,3-diaminopropionic acid (Dap), 2,4-diaminobutyric acid (Dab), ornithine, homoarginine and a combination thereof; $Xaa_{11}$ and $Xaa_{13}$ are positively charged amino acid residues.

In other embodiments, Y is a peptide chain having an amino acid sequence set forth in formula (IIa) or formula (IIb):

Xaa₇-Xaa₈-Xaa₉-Xaa₁₀-Xaa₁₁-Xaa₁₂-Xaa₁₃ formula (IIa),

Xaa₁₃-Xaa₁₂-Xaa₁₁-Xaa₁₀-Xaa₉-Xaa₈-Xaa₇ formula (IIb);

wherein, $Xaa_7$ is selected from the group consisting of L-cysteine, D-cysteine, D-homocysteine, L-homocysteine and S-methyl-D-cysteine;

Xaas is selected from the group consisting of D-alanine and D-arginine;

$Xaa_9$ is selected from the group consisting of D-arginine, D-lysine and D-histidine;

$Xaa_{10}$ is selected from the group consisting of D-arginine, D-alanine, D-lysine and D-histidine;

Xaan is selected from the group consisting of D-arginine, D-lysine and D-histidine;

$Xaa_{12}$ is D-alanine;

$Xaa_{13}$ is selected from the group consisting of D-arginine, D-lysine and D-histidine; and

the amino terminal of the peptide chain Y is free or chemically modified, and the carboxyl terminal of the peptide chain Y is free or chemically modified.

In a specific embodiment of the present disclosure, the amino acids in the peptide chain Y in the bispecific fusion polypeptide compound of the present disclosure are all D-amino acids.
In some specific embodiments of the present disclosure, the Y in the bispecific fusion polypeptide compound of the present disclosure is a polypeptide shown in SEQ ID NO: 1, Ac-D-Cys-D-Ala-D-Arg-D-Arg-D-Arg-Ala-D-Arg-NH₂ (SEQ ID NO: 1).
In some embodiments, the X in the bispecific fusion polypeptide compound of the present disclosure is a peptide chain having an amino acid sequence set forth in formula (IIIa) or formula (IIIb):

Xaa₁-Xaa₂-Xaa₃-Xaa₄-Xaa₅-Xaa₆ (IIIa),

Xaa₆-Xaa₅-Xaa₄-Xaa₃-Xaa₂-Xaa₁ (IIIb);

wherein, $Xaa_1$ is selected from the group consisting of tyrosine, homotyrosine and 3-chlorotyrosine;

$Xaa_2$ is selected from the group consisting of cysteine, homocysteine, S-methylcysteine, 2-aminoisobutyric acid, arginine, proline, glycine, homoarginine, lysine, cyclohexylalanine, sarcosine, norleucine and histidine;

$Xaa_3$ is selected from the group consisting of phenylalanine, tryptophan and tyrosine;

$Xaa_4$ is glycine, Xaas is glycine, or $Xaa_4$-$Xaa_5$ is replaced by an amino-substituted $C_{1-20}$ alkyl monocarboxylic acid;

when $Xaa_2$ is selected from the group consisting of cysteine and homocysteine, $Xaa_6$ is absent;

when $Xaa_2$ is selected from the group consisting of S-methylcysteine, 2-aminoisobutyric acid, arginine, proline, glycine, homoarginine, lysine, cyclohexylalanine, sarcosine, norleucine and histidine, $Xaa_6$ is selected from the group consisting of cysteine and homocysteine; and

the amino terminal of the peptide chain X is free or chemically modified, and the carboxyl terminal of the peptide chain X is free or chemically modified.

In other more specific embodiments, the X in the bispecific fusion polypeptide compound of the present disclosure is a peptide chain having an amino acid sequence set forth in formula (IIIa) or formula (IIIb):

$$Xaa_1\text{-}Xaa_2\text{-}Xaa_3\text{-}Xaa_4\text{-}Xaa_5\text{-}Xaa_6 \qquad (IIIa),$$

$$Xaa_6\text{-}Xaa_5\text{-}Xaa_4\text{-}Xaa_3\text{-}Xaa_2\text{-}Xaa_1 \qquad (IIIb);$$

wherein, $Xaa_1$ is selected from the group consisting of L-tyrosine, D-tyrosine, D-homotyrosine and 3-chloro-D-tyrosine;

$Xaa_2$ is selected from the group consisting of L-cysteine, D-cysteine, D-homocysteine, L-homocysteine, S-methyl-D-cysteine, 2-aminoisobutyl acid, D-arginine, L-arginine, L-proline, D-proline, glycine, D-histidine and L-cyclohexylalanine;

$Xaa_3$ is selected from the group consisting of L-phenylalanine, D-phenylalanine, D-tryptophan, L-tryptophan, D-tyrosine and L-tyrosine;

$Xaa_4$ is glycine, $Xaa_5$ is glycine, or $Xaa_4\text{-}Xaa_5$ is replaced by an amino-substituted $C_{1-20}$ alkyl monocarboxylic acid;

when $Xaa_2$ is selected from the group consisting of L-cysteine, D-cysteine, D-homocysteine and L-homocysteine, $Xaa_6$ is absent;

when $Xaa_2$ is selected from the group consisting of S-methyl-D-cysteine, 2-aminoisobutyric acid, D-arginine, L-arginine, L-proline, D-proline, glycine, D-histidine and L-cyclohexylalanine, $Xaa_6$ is selected from the group consisting of L-cysteine, D-cysteine, D-homocysteine and L-homocysteine; and

the amino terminal of the peptide chain X is free or chemically modified, and the carboxyl terminal of the peptide chain X is free or chemically modified.

In some embodiments, in the peptide chain X of the bispecific fusion polypeptide compound of the present disclosure, $Xaa_1$ is selected from the group consisting of L-tyrosine, D-tyrosine, D-homotyrosine and 3-chloro-D-tyrosine, $Xaa_2$ is selected from the group consisting of L-cysteine, D-cysteine, D-homocysteine and L-homocysteine, $Xaa_3$ is selected from the group consisting of L-phenylalanine, D-phenylalanine, D-tryptophan, L-tryptophan, D-tyrosine and L-tyrosine, $Xaa_4\text{-}Xaa_5$ is replaced by an amino-substituted $C_{1-12}$ alkyl monocarboxylic acid, and $Xaa_6$ is absent. Or in the peptide chain X, $Xaa_1$ is selected from the group consisting of L-tyrosine, D-tyrosine, D-homotyrosine and 3-chloro-D-tyrosine, $Xaa_2$ is selected from the group consisting of S-methyl-D-cysteine, 2-aminoisobutyric acid, D-arginine, L-arginine, L-proline, D-proline, glycine, D-histidine and L-cyclohexylalanine, $Xaa_3$ is selected from the group consisting of L-phenylalanine, D-phenylalanine, D-tryptophan, L-tryptophan, D-tyrosine and L-tyrosine, $Xaa_4\text{-}Xaa_5$ is replaced by an amino-substituted $C_{1-12}$ alkyl monocarboxylic acid, and $Xaa_6$ is selected from the group consisting of L-cysteine, D-cysteine, D-homocysteine and L-homocysteine.

In another embodiments, $Xaa_4\text{-}Xaa_5$ in the peptide chain X of the bispecific fusion polypeptide compound of the present disclosure is replaced by an amino-substituted $C_{1-8}$ alkyl monocarboxylic acid. In such embodiments, the expression "$Xaa_4\text{-}Xaa_5$ is replaced by an amino-substituted alkyl monocarboxylic acid" refers to that $Xaa_4\text{-}Xaa_5$ is directly replaced by an alkyl monocarboxylic acid with a substituent which is an amino. The amino-substituted alkyl monocarboxylic acid forms an amide bond with the carboxyl group of $Xaa_5$ through its amino group, and the carboxyl group in the amino-substituted alkyl monocarboxylic acid serves as the carboxyl terminal of the peptide chain X or the amino-substituted alkyl monocarboxylic acid forms an amide bond with the amino group of $Xaa_6$ through its carboxyl group.

In still another embodiments, X in the bispecific fusion polypeptide compound of the present disclosure is a cyclic peptide having a structure represented by formula (IIIc) or (IIId):

$$Cyclo\ (Xaa_1\text{-}Xaa_2\text{-}Xaa_3\text{-}Xaa_4\text{-}Xaa_5\text{-}Xaa_6) \qquad (IIIc),$$

$$Cyclo\ (Xaa_6\text{-}Xaa_5\text{-}Xaa_4\text{-}Xaa_3\text{-}Xaa_2\text{-}Xaa_1) \qquad (IIId);$$

wherein, $Xaa_1$ is selected from the group consisting of tyrosine, homotyrosine and 3-chlorotyrosine;

$Xaa_2$ is selected from the group consisting of cysteine, homocysteine, S-methylcysteine, 2-aminoisobutyric acid, arginine, proline, glycine, homoarginine, lysine, cyclohexylalanine, sarcosine, norleucine and histidine;

Xaa$_3$ is selected from the group consisting of phenylalanine, tryptophan and tyrosine;

Xaa$_4$ is glycine;

Xaas is glycine;

when Xaa$_2$ is selected from the group consisting of cysteine and homocysteine, Xaa$_6$ is absent, and the amino group of Xaa$_1$ forms a peptide bond with the carboxyl group of Xaa$_5$, or the amino group of Xaas forms a peptide bond with the carboxyl group of Xaa$_1$; and

when Xaa$_2$ is selected from the group consisting of S-methylcysteine, 2-aminoisobutyric acid, arginine, proline, glycine, homoarginine, lysine, cyclohexylalanine, sarcosine, norleucine and histidine, Xaa$_6$ is selected from the group consisting of cysteine and homocysteine, and the amino group of Xaa$_1$ forms a peptide bond with the carboxyl group of Xaa$_6$, or the amino group of Xaa$_6$ forms a peptide bond with the carboxyl group of Xaa$_1$.

In other more specific embodiments, X in the bispecific fusion polypeptide compound of the present disclosure is a cyclic peptide having a structure represented by formula (IIIc) or (IIId):

Cyclo (Xaa$_1$-Xaa$_2$-Xaa$_3$-Xaa$_4$-Xaa$_5$-Xaa$_6$)          (IIIc),

Cyclo (Xaa$_6$-Xaa$_5$-Xaa$_4$-Xaa$_3$-Xaa$_2$-Xaa$_1$)          (IIId);

wherein, Xaa$_1$ is selected from the group consisting of L-tyrosine, D-tyrosine, D-homotyrosine and 3-chloro-D-tyrosine ;

Xaa$_2$ is selected from the group consisting of L-cysteine, D-cysteine, D-homocysteine, S-methyl-D-cysteine, 2-aminoisobutyric acid, D-arginine, L-arginine, glycine and D-proline;

Xaa$_3$ is selected from the group consisting of D-phenylalanine, L-phenylalanine, D-tryptophan and D-tyrosine;

Xaa$_4$ is glycine;

Xaas is glycine;

when Xaa$_2$ is selected from the group consisting of L-cysteine, D-cysteine and D-homocysteine, Xaa$_6$ is absent, and the amino group of Xaa$_1$ forms a peptide bond with the carboxyl group of Xaa$_5$, or the amino group of Xaa$_5$ forms a peptide bond with the carboxyl group of Xaa$_1$; and

when Xaa$_2$ is selected from the group consisting of S-methyl-D-cysteine, 2-aminoisobutyric acid, D-arginine, L-arginine, glycine and D-proline, Xaa$_6$ is selected from the group consisting of L-cystine, D-cysteine and D-homocysteine, and the amino group of Xaa$_1$ forms a peptide bond with the carboxyl group of Xaa$_6$, or the amino group of Xaa$_6$ forms a peptide bond with the carboxyl group of Xaa$_1$.

The cyclic peptide X in the bispecific fusion polypeptide compound of the present disclosure can effectively resist enzymatic degradation, thereby improving stability, which further improves bioavailability and metabolic stability.

In some specific embodiments of the present disclosure, X in the bispecific fusion polypeptide compound of the present disclosure is a peptide chain selected from the group consisting of SEQ ID NOs: 2-6 and SEQ ID NOs: 12-18:

(1) SEQ ID NO: 2
H$_2$N-L-Tyr-L-Cys-L-Phe-Gly-Gly-OH;

(2) SEQ ID NO: 3
H$_2$N-D-Tyr-L-Cys-D-Phe-Gly-Gly-OH;

(3) SEQ ID NO: 4
H$_2$N-D-Tyr-D-Cys-D-Phe-Gly-Gly-OH;

(4) SEQ ID NO: 5
$H_2N$-D-Tyr-Aib-D-Phe-Gly-Gly-L-Cys-OH;

(5) SEQ ID NO: 6
$H_2N$-D-Tyr-D-Arg-D-Phe-Gly-Gly-L-Cys-OH;

(6) SEQ ID NO: 12
$H_2N$-D-Tyr-D-His-D-Tyr-Gly-Gly-L-Cys-OH;

(7) SEQ ID NO: 13
$H_2N$-D-Tyr-D-Pro-D-Phe-Gly-Gly-L-Cys-OH;

(8) SEQ ID NO: 14
Cyclo (D-Tyr-Aib-D-Phe-Gly-Gly-L-Cys);

(9) SEQ ID NO: 15
Cyclo (D-Tyr-D-Arg-D-Phe-Gly-Gly-L-Cys);

(10) SEQ ID NO: 16
Cyclo (L-Tyr-Gly-L-Phe-Gly-Gly-L-Cys);

(11) SEQ ID NO: 17
Cyclo (D-Tyr-Gly-D-Phe-Gly-Gly-D-Cys); and

(12) SEQ ID NO: 18
Cyclo (D-Cys-Gly-Gly-D-Phe-Gly-D-Tyr).

In some embodiments, ID in the bispecific fusion polypeptide compound of the present disclosure is an intramolecular disulfide bond or a linker between X and Y;

the intramolecular disulfide bond is formed between cysteine, homocysteine or S-methylcysteine in X and cysteine, homocysteine or S-methylcysteine in Y; and

the linker is selected from the group consisting of an amino-substituted $C_{1-8}$ alkyl acid, a polyethylene glycol polymer chain and a peptide segment consisting of 1-10 amino acids, and the amino acid in the peptide segment is selected from the group consisting of proline, arginine, alanine, threonine, glutamic acid, aspartic acid, lysine, glutamine, asparagine and glycine. In some specific embodiments of the present disclosure, the linker is selected from the group consisting of: (1) (Gly-Ser)$_n$, wherein n is 0, 1, 2 or 3; (2) Gly-Ser-Gly; (3) Ser-Gly-Gly-Ser-Gly-Gly-Ser; (4) 4-aminobutyric acid or 6-aminocaproic acid; and (5) (PEG)$_m$, wherein m is 1, 2, 3, 4, or 5.

In some embodiments, in the bispecific fusion polypeptide compound of the present disclosure, the peptide may be modified at the N-terminal (amino-terminal), C-terminal (carboxy-terminal), or both. The chemical modification of the amino terminal of the peptide chain X or the peptide chain Y includes acylation, sulfonylation, alkylation and PEGylation modification, and the chemical modification of the carboxyl terminal of the peptide chain X or the peptide chain Y includes amidation, sulfonylation and PEGylation. In some specific embodiments of the present disclosure, the chemical modification of the amino terminal is acetylation, benzoylation and sulfonylation of the amino group, and the alkylation of the amino terminal is $C_{1-6}$ alkylation or aralkylation. The chemical modification of the carboxyl terminal is that the OH in the carboxyl group is substituted by $NH_2$ or sulfonamide. In some specific embodiments of the present disclosure, the N-terminal and C-terminal are modified by acetylation and amidation, respectively.

In specific embodiments of the present disclosure, the bispecific fusion polypeptide compound represented by formula (Ia) or formula (Ib) is selected from the group consisting of SEQ ID NOs: 7-11 and SEQ ID NOs: 19-27, or a pharmaceutically acceptable salt thereof.

(1) SEQ ID NO: 7

(2) SEQ ID NO: 8

(3) SEQ ID NO: 9

(4) SEQ ID NO: 10

(5) SEQ ID NO: 11

(6) SEQ ID NO: 19

(7) SEQ ID NO: 20

Ac —— D-Cys —— D-Arg —— D-Arg —— D-Ala —— D-Arg —— D-Ala —— D-Arg —— NH₂

S —— S

H₂N —— D-Tyr —— Aib —— D-Phe —— Gly —— Gly —— L-Cys —— OH

(8) SEQ ID NO: 21

Ac —— D-Cys —— D-Ala —— D-Arg —— D-Arg —— D-Arg —— D-Ala —— D-Arg —— NH₂

S —— S

H₂N —— D-Tyr —— D-His —— D-Tyr —— Gly —— Gly —— L-Cys —— OH

(9) SEQ ID NO: 22

Ac —— D-Cys —— D-Ala —— D-Arg —— D-Arg —— D-Arg —— D-Ala —— D-Arg —— NH₂

S —— S

H₂N —— D-Tyr —— D-Pro —— D-Phe —— Gly —— Gly —— L-Cys —— OH

(10) SEQ ID NO: 23

Ac —— D-Cys —— D-Ala —— D-Arg —— D-Arg —— D-Arg —— D-Ala —— D-Arg —— NH₂

S —— S

Cyclo ( D-Tyr —— Aib —— D-Phe —— Gly —— Gly —— L-Cys )

(11) SEQ ID NO: 24

Ac —— D-Cys —— D-Ala —— D-Arg —— D-Arg —— D-Arg —— D-Ala —— D-Arg —— NH₂

S —— S

Cyclo ( D-Tyr —— D-Arg —— D-Phe —— Gly —— Gly —— L-Cys )

(12) SEQ ID NO: 25

Ac —— D-Cys —— D-Ala —— D-Arg —— D-Arg —— D-Arg —— D-Ala —— D-Arg —— NH₂

S —— S

Cyclo ( L-Tyr —— Gly —— L-Phe —— Gly —— Gly —— L-Cys )

(13) SEQ ID NO: 26

```
Ac —— D-Cys —— D-Ala —— D-Arg —— D-Arg —— D-Arg —— D-Ala —— D-Arg —— NH₂
              S —— S
Cyclo ( D-Tyr —— Gly —— D-Phe —— Gly —— Gly —— D-Cys)
```

(14) SEQ ID NO: 27

```
Ac —— D-Cys —— D-Ala —— D-Arg —— D-Arg —— D-Arg —— D-Ala —— D-Arg —— NH₂
        S —— S
Cyclo ( D-Cys —— Gly —— Gly —— D-Phe —— Gly —— D-Tyr)
```

In the above structure, "Ac" represents an acetyl capping group, and "NH₂" represents an amide capping group.

The bispecific fusion polypeptide compounds of the present disclosure also include a derivative obtained by chemical modification on the side chain group of amino acid of the polypeptide compounds The derivatives are thioethers or glucosinolates formed by the thiol group of cysteine in the polypeptide compound, or a compound containing a disulfide bond formed with cysteine or a cysteine-containing peptide; or

the derivatives are an ester, ether or glycoside compound formed by the phenolic hydroxyl group of tyrosine in the polypeptide compound; or

the derivatives are a complex or chelate formed by the polypeptide compound and a metal ion;

the derivatives are a derivative formed after the phenyl ring of tyrosine and phenylalanine is substituted; or

the derivatives are a hydrate or solvate formed by the polypeptide compound.

It should be noted that other variants of the bispecific fusion polypeptide compound disclosed in the present disclosure are also included in the scope of the present disclosure, and specifically included are any variants obtained by substitution of conserved amino acids only.

The bispecific fusion polypeptide compound provided by the present disclosure may exist in the form of a free polypeptide or in the form of a salt. In some embodiments, the salt refers to a pharmaceutically acceptable salt. The "pharmaceutically acceptable salt" of the present disclosure may be synthesized from the parent compound and basic or acidic moiety by conventional chemical methods. In general, such salt can be prepared by reacting the free acid form of these compounds with a stoichiometric amount of a suitable base (e.g., hydroxide, carbonate, bicarbonate of Na, Ca, Mg, or K, and the like), or by reacting the free base form of these compounds with a stoichiometric amount of an appropriate acid.

The bispecific fusion polypeptide compound provided by the present disclosure is constructed by connecting structures, and belongs to the active polypeptide with multi-target, which can not only activate calcium-sensing receptor (CasR), but also function as an osteogenic growth peptide.

Compared with single-target peptides, the active polypeptide compounds constructed based on the dual-target effects can exert multiple effects of (1) activating calcium-sensing receptors, reducing plasma parathyroid hormone (PTH) levels through intracellular signaling pathways, activating the mitogen-activated protein kinase (MAPK) pathway mediated by calcium-sensing receptors on the surface of osteoblasts, thereby stimulating the proliferation and differentiation of osteoblasts, inhibiting the bone resorption of osteoclasts by causing the differentiation and apoptosis of osteoclasts; and (2) activating osteogenic growth peptide receptors, stimulating the proliferation of osteoblasts and fibroblasts, promoting bone calcification, and promoting bone formation.

The present disclosure also provides uses of the bispecific fusion polypeptide compounds and the pharmaceutical composition in the manufacture of a medicament. The bispecific fusion polypeptide compounds provided by the present disclosure can be used for preventing and/or treating a disease selected from the group consisting of an elevated parathyroid hormone disease, malignant hypercalcemia, metastasis bone disease, Paget's disease, osteoarthritis, rheumatoid arthritis, osteomalacia, chondro-calcinosis articularis, achondroplasia, osteochondritis, cystic osteogenesis imperfecta, congenital hypophosphatasia, fibromatous lesion, fibrous dysplasia, multiple myeloma, osteolytic bone disease, periprosthetic osteolysis, periodontal disease, osteoporosis, abnormal bone turnover, high turnover bone disease, chronic kidney disease-mineral, bone disorder (CKD-MBD) and a combination thereof. The elevated parathyroid hormone disease is preferably primary hyperparathyroidism, secondary hyperparathyroidism and tertiary hyperparathyroidism. The secondary hyperparathyroidism is preferably elevated parathyroid hormone caused by chronic kidney disease hemodialysis and/or peritoneal dialysis.

The bispecific fusion polypeptide compounds provided by the present disclosure can significantly reduce the normal parathyroid hormone (PHT) level, increase the serum phosphorus level, and reduce the serum calcium level, which can be used for the prevention and/or treatment of hypercalcemia and hypophosphatemia, and/or diseases caused by elevated parathyroid hormone.

The present disclosure provides a method for treating, i.e., ameliorating, or preventing hyperparathyroidism associated with chronic kidney disease (i.e., stages 1-4) by reducing blood PTH level in a subject with this disease. The method comprises administering the compounds to a subject in need thereof to reduce blood PTH level to suppress parathyroid gland activity.

The drugs used in the pharmacodynamic activity test are a polypeptide compound having an amino acid sequence set forth in any one of SEQ ID NOs: 7-11.

In another aspect, the present disclosure also provides a pharmaceutical composition comprising the bispecific fusion polypeptide compounds of the present disclosure. Optionally, the pharmaceutical composition further comprises at least one of a pharmaceutically acceptable adjuvant, excipient, carrier and solvent.

As used herein, the term "pharmaceutically acceptable" means that the substance or composition must be chemically and/or toxicologically compatible with the other ingredients contained in the formulation and/or the mammal to be treated.

As used herein, the term "pharmaceutically acceptable excipient" means a pharmaceutically acceptable material, mixture or vehicle that is relevant to the consistency of the administered dosage form or pharmaceutical composition. Each excipient must be compatible with the other ingredients of the pharmaceutical composition when mixed, to avoid interactions that would greatly reduce the efficacy of the compounds disclosed in the present disclosure when administered to patients and interactions that would lead to the pharmaceutical composition pharmaceutically unacceptable.

Unless otherwise specified, all technical and scientific terms used in the present disclosure have the same meaning as those generally understood by those skilled in the art to which the present disclosure belongs. All patents and publications related to the present disclosure are incorporated by reference in their entirety.

The term "optional" or "optionally" means that the subsequently described event or environment may but does not necessarily occur, and the description includes the scenarios where the event or environment occurs and the scenarios where the event or environment does not occur.

The term "natural amino acid" refers to all 20 amino acids that naturally exist. The term "unnatural amino acid" refers to D-amino acids corresponding the L-amino acids , such as D-Ala for L-Ala, etc., or suitable modifications of L- or D-amino acids and aminoalkyl acids by the following routes.

The absolute "S" configuration on the "alpha" carbon is often referred to as the "L" or native configuration. The "R" configuration on the "alpha" carbon is often referred to as a "D" amino acid. Where two "alpha-substituents" (e.g., hydrogen or methyl) are the same, the amino acid is Gly or Aib and is not chiral.

As used herein, "amino acid" refers to both natural and unnatural amino acids. The stereoconfiguration of amino acids is indicated by a three-letter code with the prefix "L-" or "D-" (except for achiral glycines), for example, amino acids in the L configuration include: alanine ("L-Ala" or "A"), arginine ("L-Arg" or "R"), asparagine ("L-Asn" or "N"), aspartic acid ("L-Asp" or "D"), cysteine ("L-Cys" or "C"), glutamine ("L-Gln" or "Q"), glutamic acid ("L-Glu" or "E"), glycine ("Gly" or "G"), histidine ("L-His" or "H"), isoleucine ("L-Ile" or "I"), leucine ("L-Leu" or "L"), lysine ("L-Lys" or "K"), methionine ("L-Met" or "M"), phenylalanine ("L-Phe" or "F"), proline ("L-Pro" or "P"), serine ("L-Ser" or "S"), threonine ("L-Thr" or "T"), tryptophan ("L-Trp" or "W"), tyrosine ("L-Tyr" or "Y"), and valine ("L-Val" or "V"). L-norleucine and L-norvaline can be represented as (NLeu) and (NVal), respectively. Nineteen naturally occurring chiral amino acids have corresponding D-isomers, which are indicated by a three-letter code with the prefix "D-": alanine ("D-Ala" or "a"), arginine acid ("D-Arg" or "r"), asparagine ("D-Asn" or "a"), aspartic acid ("D-Asp" or "d"), cysteine ("D-Cys" or "c"), glutamine ("D-Gln" or "q"), glutamic acid ("D-Glu" or "e"), histidine ("D-His" or "h"), isoleucine ("D-Ile" or "i"), leucine ("D-Leu" or "l"), lysine ("D-Lys" or "k"), methionine ("D-Met" or "m"), phenylalanine ("D-Phe" or "f"), proline ("D-Pro" or "p"), serine ("D-Ser" or "s"), threonine ("D-Thr" or "t"), tryptophan ("D-Trp" or "w"), tyrosine ("D-Tyr" or "y") and valine ("D-Val" or "v").

Regarding the representation of the amino acids of the present disclosure, when the configuration is not specified, the amino acids only represented by the amino acid name or three-letter code include L-isomers and D-isomers. When the name of an unnatural amino acid is preceded by the prefix "L" or "D", it refers to the single configuration identified. For example: "alanine" or "Ala" represents D-Ala and L-Ala; "cysteine" or "Cys" represents D-Cys and L-Cys; and homocysteine represents D-Homocysteine and L-homocysteine.

The terms "amino acid" and "amino acid residue" are used interchangeably in the present disclosure, although "amino acid residue" is usually used in reference to a subunit of peptide, polypeptide or protein monomer, and "amino acid" is usually used in reference to a free molecule.

Every two amino acids are connected to each other to form a peptide bond, and multiple amino acids are connected to each other to form multiple peptide bonds. A chain structure containing multiple peptide bonds formed by the connection of multiple amino acids is called "peptide chain" or ""peptide segment". As used herein, "peptide" and "polypeptide" refer to a polymer composed of chains of amino acid residues connected by peptide bonds, regardless of molecular size. The terms "peptide" and "polypeptide" are used interchangeably in the present disclosure.

Unless otherwise indicated, peptide sequences are given in order from amino-terminal (N-terminal) to carboxy-terminal (C-terminal).

The term "linker" used in the present disclosure is a linking fragment used to connect the polypeptide fragment X and the polypeptide fragment Y, as long as it has no effect on the physiological activity of the peptide chain X and the peptide chain Y, and there is no limitation on its length and structure. The linker can provide a certain space for the two peptides to fold correctly without interfering with each other. The linker also provides more possibilities for the two peptides to interact and promote mutual synergy. Linkers include hydrophobic linkers, flexible hydrophilic linkers and peptide fragment linkers. The hydrophobic linker in the present disclosure is mainly aminoalkanoic acids, such as 4-aminobutyric acid or 6-aminocaproic acid; the commonly used hydrophilic linker is a PEG polymer, such as $(PEG)_m$, where m is 1, 2, 3, 4 or 5; a peptide linker is a peptide fragment consisting of 1-10 amino acids. Considering the convenience of preparation, etc., the linker is a polypeptide fragment with a length of 1-10 amino acids containing an enzyme cleavage site. In some embodiments, the linker is a fragment with a length of 2-8 amino acids; in some embodiments, the linker length is a fragment of 3 amino acids. In an embodiment of the present disclosure, the amino acids that make up the linker are selected from the group consisting of proline, arginine, phenylalanine, threonine, glutamic acid, aspartic acid, lysine, glutamine, asparagine and glycine. In the actual preparation example of the present disclosure, the linker is L-Gly-L-Ser-L-Gly, $(L-Gly-L-Ser)_2$, $(L-Gly-L-Ser)_3$ or L-Ser-L-Gly-L-Gly-L-Ser-L-Gly-L-Gly-L-Ser. The linker separates the two partial peptide chains to reduce the steric hindrance effect between each other, and the linker can be hydrolyzed in the body, which is beneficial for each peptide segment to exert an active effect.

The term "calcium-sensing receptor agonist" used in the present disclosure, also known as "calcimimetic", calcium-sensing receptor activator, refers to a substance that can bind to the extracellular domain (ECD), transmembrane domain or intracellular domain (ICD) of the calcium-sensing receptor (CaSR) to activate the CaSR expressed on the cell surface of parathyroid cells, thereby activating cell signaling pathways to achieve inhibition of PTH synthesis and secretion. Calcium-sensing receptor agonists may be small molecule compounds or polypeptide molecules. Calcium-sensing receptor agonists include, but are not limited to, the first generation of phenylalkylamine calcium-sensing receptor agonists represented by NPS R-568 and NPS R-467, the phenylalkylamine calcium-sensing receptor agonists represented by cinacalcet, the third generation of polypeptide calcium-sensing receptor agonists represented by etelcalcetide, and other active polypeptides with similar activities, such as the peptide chain Y of the compound of the present disclosure.

Osteogenic growth peptide (OGP)-like peptide (osteogenic growth peptide receptor activator) refers to a substance that can activate the osteogenic growth peptide receptor signaling pathway, promote the proliferation and differentiation of osteoblasts, stimulate the division and proliferation of bone marrow hematopoietic stem cells and bone marrow mesenchymal stem cells, and can maintain self-recovery ability of hematopoietic stem cells and inhibit megakaryocyte growth. Osteogenic growth peptide can activate MAP kinase, Src and RhoA pathways. Activation of the MAP pathway will increase mitosis and promote the division and proliferation of osteoblasts, bone marrow hematopoietic stem cells and bone marrow mesenchymal stem cells. Activation of the Src and RhoA pathways can regulate the autocrine expression of endogenous osteogenic growth peptide in osteoblasts, promote the secretion of alkaline phosphatase, up-regulate the transcription of type I collagen, osteocalcin, and Cbfα1 mRNA, promote calcium salt deposition and matrix mineralization, promote osteogenesis, accelerate fracture healing, and increase bone density. Osteogenic growth peptide-like peptides include, but are not limited to, immunoreactive OGP, specifically including free OGP, OGP (10-14), recombinant OGP and OGP binding protein (OGPBP), and natural or synthetic polypeptide compounds with similar activity, such as the peptide chain X of the compound of the present disclosure.

Bone marrow mesenchymal stem cells (BMSCs), also known as bone marrow stromal cells, not only provide mechanical support for hematopoietic stem cells (HSCs) in the bone marrow, but also secrete a variety of cytokines that regulate hematopoiesis (such as IL-6, IL-11, LIF, M-CSF and SCF, etc.) to support hematopoiesis. In addition, they have the potential to differentiate into osteoblasts, fibroblasts, reticulocytes, adipocytes and endothelial cells. Bone marrow mesenchymal stem cell stimulators refer to substances that can stimulate BMSCs to secrete cytokines that regulate hematopoiesis, thereby promoting hematopoietic function, and/or can induce BMSCs to proliferate and differentiate. Bone marrow mesenchymal stem cell stimulators include, but are not limited to, immunoreactive OGP, specifically including free OGP, OGP (10-14), recombinant OGP and OGP binding protein (OGPBP), and natural or synthetic polypeptide compounds with similar activity, such as the peptide chain X in the compound of the present disclosure.

The term "alkyl group" or "alkyl" used herein means a saturated linear or branched monovalent hydrocarbon group containing 1 to 20 carbon atoms, wherein the alkyl group may be optionally substituted with one or more substituents described herein. Unless otherwise specified, alkyl groups contain 1-20 carbon atoms. In some embodiments, the alkyl group contains 1-12 carbon atoms. In some other embodiments, the alkyl group contains 2-12 carbon atoms. In some other embodiments, the alkyl group contains 1-6 carbon atoms. In some other embodiments, the alkyl group contains 2-6 carbon atoms. In still other embodiments, the alkyl group contains 1-4 carbon atoms. In still other embodiments, the alkyl group contains 1-3 carbon atoms. The alkyl group may be optionally substituted with one or more substituents described herein.

As used herein, "thiol-containing group" or "thiol-containing moiety" means a functional group that contains a sulfur-hydrogen bond (-SH) and is capable of reacting with another thiol group to form a disulfide bond under physiological conditions. In the present disclosure, a thiol group capable of forming a disulfide bond with another thiol group is referred to as "active thiol group".

Peptide therapeutics are susceptible to attack by peptidases. Exopeptidases are typically nonspecific enzymes that cleave amino acid residues from the amino or carboxy terminal of peptides or proteins. Endopeptidases that cleave within amino acid sequences may also be nonspecific; however, endopeptidases often recognize specific amino sequences (recognition sites) and cleave peptides at or near those sites. Therefore, modification of the compound to protect it from proteolytic degradation is contemplated. One approach to protecting peptides from proteolytic degradation involves chemical modification, or "capping" the amino and/or carboxyl terminal of peptides.

In some embodiments, the N-terminal and C-terminal of the peptides of the present disclosure may be free. When the C-terminal is free, no substituent is shown or it is represented by "-OH", and when the N-terminal is free, no substituent is shown or it is represented by "H". In other specific embodiments, the peptide segment of the present disclosure may be chemically modified.

As used herein, the term "chemical modification" or "capping" are used interchangeably and refers to the introduction of a protecting group to one or both ends of a compound via covalent modification. Suitable protecting groups serve to cap the ends of the peptide without reducing the biological activity of the peptide. Any residue located at the amino or carboxy terminal, or both, of the compounds can be chemically modified, including thiol-containing amino acids.

In some embodiments, the amino terminal of the compound is chemically modified by acetylation to produce an N-acetyl peptide (which may be represented as "Ac-" in a structure or formula of the present disclosure). In some embodiments, the carboxy terminal of the peptide is chemically modified by amidation to produce a primary carboxamide at the C-terminal (which may be represented as "-NH$_2$" in the peptide sequence, structure or formula of the present disclosure). In some embodiments, the amino-terminal and carboxy-terminal are chemically modified by acetylation and amidation, respectively. However, other capping groups are possible. For example, the amino terminal may be capped by acylation with groups such as acetyl, benzoyl, etc., or with natural or unnatural amino acids, such as acetyl-capped β-alanine, or capped by alkylation with groups such as benzyl or butyl, or capped by sulfonylation to form sulfonamide. Similarly, the carboxy terminal may be esterified or converted to secondary amides and acylsulfonamides and the like. In some embodiments, the amino-terminal or carboxyl-terminal may contain a site for the attachment of a polyethylene glycol (PEG) moiety, that is, the amino- or carboxyl-terminal may be chemically modified by reaction with a suitable functionalized PEG. "PEG" means polyethylene glycol.

The "elevated parathyroid hormone disease" used herein refers to a disease with elevated parathyroid hormone level higher than the normal range, caused by chronic kidney disease hemodialysis and/or peritoneal dialysis. The main function of parathyroid hormone (PTH) is to regulate the metabolism of calcium and phosphorus in vertebrates, promote the increase of blood calcium level and reduce the blood phosphorus level. Disorders associated with elevated PTH include hypercalcemia, hypophosphatemia, skeletal decalcification, urinary stones, kidney damage, decreased neuromuscular excitability, cardiovascular system disease, etc. caused by hyperparathyroidism and elevated parathyroid hormone levels.

The term "hyperparathyroidism" refers to primary, secondary and tertiary hyperparathyroidism. Hyperparathyroidism is subdivided into primary, secondary and tertiary hyperparathyroidism. In primary hyperparathyroidism, the growth of the parathyroid glands is virtually autonomous, usually due to tumors such as parathyroid adenomas, and may be irreversible. The adenomas usually do not display resistance to vitamin D receptors but display resistance to the natural hormone form of vitamin D, 1,25-dihydroxyvitamin D$_3$. In secondary hyperparathyroidism, parathyroid hyperplasia associated with, for example, 1,25-dihydroxyvitamin D deficiency and/or resistance is usually adaptive due to resistance to the effects of hormone metabolism, and may be reversible. Secondary hyperparathyroidism occurs in patients with, for example, nephropathy, osteomalacia, and intestinal malabsorption syndrome.

Tertiary hyperparathyroidism is characterized by a state of autonomous proliferation of the biologically hyperparathyroid glands. Tertiary hyperparathyroidism may occur in patients with secondary hyperparathyroidism, in which the reversible hyperplasia associated with secondary hyperparathyroidism changes into an irreversible growth defect, and the enlarged tissues have vitamin D receptors. In all forms of hyperparathyroidism, bone abnormalities such as bone loss or decreased mineral content are prevalent, and kidney damage may be present. Therefore, hyperparathyroidism is also characterized by abnormal calcium, phosphorus and bone metabolism.

The compounds of the present disclosure may be administered as the sole active ingredient, or may be administered in combination with other therapeutic ingredients, including other compounds having the same or similar therapeutic activity and determined to be safe and effective for such combination administration.

The compounds of the present disclosure may be administered as a separate active ingredient, or may be administered in combination with other therapeutic drugs, including other compounds that have the same or similar therapeutic activity and are determined to be safe and effective for such combined administration.

## BRIEF DESCRIPTION OF DRAWINGS

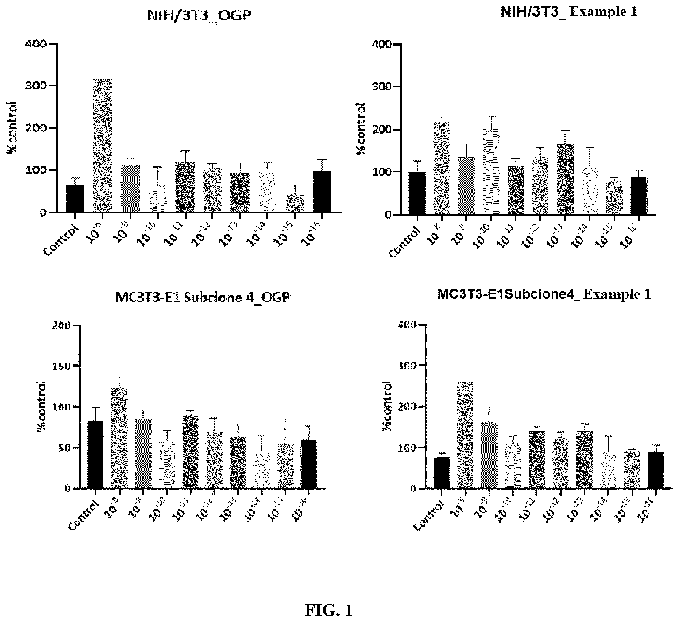

FIG. 1. *In vitro* proliferation effect of the polypeptide of Example 1 on osteoblast MC3T3-E1 Subclone 4 and fibroblast NIH/3T3 cells.

## DETAILED DESCRIPTION

The present disclosure will be illustrated in further detail below with reference to specific embodiments, but the embodiments of the present disclosure are not limited thereto. The embodiments of the present disclosure are only given to illustrate the present disclosure, rather than to limit the present disclosure. Therefore, any improvement to the present disclosure under the premise of the method of the present disclosure belongs to the protection scope of the present disclosure. Generally, the compounds of the present disclosure can be prepared by the methods described in the present disclosure. Those skilled in the art can also use well-known methods to select sequential or different synthetic steps to produce polypeptide compounds having the structures of the present disclosure. The following reaction schemes and examples serve to further illustrate the content of the present disclosure.

The polypeptide compounds of the present disclosure may be prepared by recombinant or synthetic methods. In the embodiments of the present disclosure, the artificially synthesized polypeptide compounds have the physiological effect described in the present disclosure.

Those skilled in the art will recognize that the polypeptide compounds described herein can be prepared by solid-phase peptide synthesis (SPPS), liquid phase synthesis and enzymatic synthesis. The polypeptide compounds of the present disclosure prepared by different preparation methods all fall within the scope of the present disclosure. For example, polypeptide compounds are usually prepared by solid-phase synthesis. In the solid-phase synthesis, conventional polystyrene-divinyl-benzen cross-linked resins, polyacrylamide, polyethylene-glycol resins, etc., may be used, for example, Wang Resin, Fmoc-Pro-CTC, Rink Amide Linker MBHA resin, etc. According to different connecting sequences, the appropriate resin is selected.

For example, the carboxyl group of the carboxyl-terminal amino acid can be covalently linked to the polymer solid-phase carrier, and the α-amino group is protected by the protecting group selected from Fmoc, Boc and CBz. From the C-terminal to the N-terminal, a peptide chain resin with a protecting group is obtained through the repeated process of deprotection, condensation, re-deprotection, and condensation in a given order, and then the desired peptide segment is obtained by cleaving the resin and removing the protecting group. It is also possible to covalently link the amino group of the amino-terminal amino acid to the polymer solid-phase carrier. Through the reverse sequence synthesis, a peptide chain resin with a protecting group is obtained through the repeated process of deprotection, condensation, re-deprotection, and condensation in a given order, and then the desired peptide segment is obtained by cleaving the resin and removing the protecting group. When preparing a polypeptide compound by solid-phase synthesis, depending on the type of resin selected, either a polypeptide with a free carboxyl terminal or a peptide chain modified by amidation at the carboxyl terminal may be obtained.

In the present disclosure, a peptide chain may be synthesized on an insoluble polymer support (resin) starting from the C-terminal of the peptide in a stepwise fashion. In some embodiments, synthesis is initiated by attaching the C-terminal amino acid of the peptide to the resin by forming an amide, ester, or ether bond. This approach allows the final release of a peptide chain with amide, carboxylic acid or alcohol at C-terminal, respectively.

In Fmoc-based SPPS, differential protection (orthogonal protection) is required for the α-amino and side chain functional groups (if present) of the C-terminal amino acid and all other amino acids used in the synthesis, so that the α-amino protecting group can be selectively removed during the synthesis using a suitable base (e.g., 20% piperidine solution) without premature cleavage of the peptide from the resin or deprotection of side chain protecting groups (usually acid-sensitive protecting groups for protection). Coupling of amino acids is carried out by activating the carboxyl group of the amino acid into an active ester and reacting it with the unblocked α-amino group of the amino acid attached to the N-terminal of the resin. After each coupling and deprotection, the peptide-resin is washed with excess solvent (e.g., DMF, DCM and ether). The sequential α-amino deprotection and coupling are repeated until the desired peptide sequence is synthesized. The peptide is then cleaved from the resin using a suitable cleavage buffer (usually in the presence of a suitable scavenger to limit side chain reactions) while the side chain functionalities are deprotected. The resulting peptides are subjected to final purification by reverse phase HPLC.

When identifying the structure of polypeptide compounds, QE identification analysis, mass spectrometry protein N-terminal sequence analysis, and polypeptide protein N-terminal sequence analysis are used to confirm the primary structure, and circular dichroism scanning analysis is used to determine the secondary structure.

In the QE identification analysis of the polypeptide compounds of the examples of the present disclosure, endoproteinase (usually trypsin) is used to perform enzymatic hydrolysis on protein polypeptide samples, and then by LC/MS/MS (nanoLC-QE), the enzymatically hydrolyzed samples are analyzed. Finally, the LC/MS/MS data are analyzed using mass spectrometry matching software such as MASCOT to obtain the qualitative identification information of the target protein and polypeptide molecules.

The experimental method used in the mass spectrometry protein N-terminal sequence analysis of the polypeptide compounds of the examples of the present disclosure is to use trypsin, chymotrypsin and Glu-C enzyme to perform enzymatic hydrolysis on the protein, and then by LC-MS/MS (Xevo G2-XS QTof, waters), the enzymatically hydrolyzed peptide samples are analyzed.

The N-terminal sequence analysis of the polypeptide compounds of the examples of the present disclosure is to analyze the N-terminal sequence of the test sample by an automatic protein polypeptide sequencer. The following are set by the software PPSQ Analysis: sample name, sample number, number of test cycles and selection method file, and the test is started after the setting is completed. Data and spectrum processing: the raw data and spectrum generated by PPSQ are identified by PPSQ DataProcessing software to identify peaks and export the corresponding spectrum.

The preliminary structure of the polypeptide compounds of the examples of the present disclosure is determined by mass spectrometry, and the relative molecular mass of the protein is tested by high-resolution mass spectrometry, so as to obtain the relative molecular mass information of the polypeptide accurately and reliably.

The following abbreviations are used throughout this disclosure:

r.t.: Room temperature

DIEA: Diisopropylethylamine

$H_2O$: Water

$CH_3CN$: Acetonitrile

DMF: N,N-Dimethylformamide

HBTU: 2-(1H-benzotriazol-1-yl-)-1,1,3,3-tetramethylurea hexafluorophosphate

Fmoc: 9H-fluoren-9-ylmethoxycarbonyl

Boc: tert-butoxycarbonyl

t-Bu: tert-butyl

Trt: Trityl

DCM: Dichloromethane

TIS: Triisopropylsilane

TFA: Trifluoroacetic acid

$Et_2O$: Diethyl ether

NMP: 1-Methyl-pyrrolidin-2-one

DIEA: Diisopropylethylamine

EDT: Ethanedithiol

TA: Thioanisole

PyBOP: 1H-benzotriazol-1-yloxytripyrrolidinyl hexafluorophosphate

**Preparation Examples**

**Example 1: Preparation of Active Polypeptide (SEQ ID NO: 10)**

Ac——D-Cys——D-Ala——D-Arg——D-Arg——D-Arg——D-Ala——D-Arg——NH$_2$

S——S

H$_2$N——D-Tyr——Aib——D-Phe——Gly——Gly——L-Cys——OH

(1) The peptide fragment Ac-D-Cys-D-Ala-D-Arg-D-Arg-D-Arg-D-Ala-D-Arg-NH$_2$ (peptide sequence shown in SEQ ID NO: 1) was prepared by synthesizing from C-terminal to N-terminal.

(1-1) Pretreatment of resin: Rink Amide Resins were weighed and put into a 250 mL reaction tube, swollen with DCM, washed with DMF. Piperidine/DMF solution was added for deprotection. The resins were washed with DMF and dried. The resins were tested by ninhydrin for color and the resins were purple-black.

(1-2) Coupling S1 amino acid: Fmoc-D-Arg(pbf)-OH and PyBOP were weighed and dissolved in DMF, and added into DIEA. Nitrogen was introduced, the mixture was stirred and reacted for 1.5-3 hours. After the reaction, the resins were detected with ninhydrin. If the resins were colorless and transparent, the reaction was completed. After the solvent was drained, the resins were washed with DMF, and piperidine/DMF solution was added for deprotection for 20-30 min. After the deprotection, the reagent was drained. The resins were washed with DMF and the solvent was drained. The resins were then subjected to ninhydrin test. When the color of the resins was purplish-black, the next step was carried out.

(1-3) Fmoc-D-Ala-OH was weighed and linked by step (1-2) as S2 amino acid.

(1-4) Fmoc-D-Arg(pbf)-OH was weighed and linked by step (1-2) as S3 amino acid. Step (1-4) was then repeated twice to link Fmoc-D-Arg(pbf)-OH as S4 and S5 amino acids.

(1-5) Fmoc-D-Ala-OH was weighed and linked by step (1-2) as S6 amino acid.

(1-6) Fmoc-D-Cys(trt)-OH was weighed and linked as S7 amino acid.

(1-7) N-terminal acetylation: DMF was cooled to 5-15°C, acetic anhydride and pyridine were added to prepare a mixed solvent (1). The resins obtained in step (1-6) were cooled and added into the mixed solvent (1). After the addition, the reaction was performed at a controlled temperature for 1-2 hours. After the color of the resins tested by ninhydrin was colorless and transparent, the solvent was drained. The resins were then washed with DMF, and the solvent was drained.

(1-8) Resin washing: the resins were washed first with methanol and then DCM, and repeated 2-4 times, then put into a vacuum oven for drying.

(1-9) Cleavage of peptide: 155 mL of TFA, 8 mL of TIS (triisopropyl silane), 4.12 mL of EDT (ethanedithiol), 2 mL of TA (thioanisole), 4.12 mL of water, and 2 mL of anisole were mixed evenly to prepare a lysis buffer. After the peptide fragment was cleaved, the buffer was removed by filtration under reduced pressure with a sand core funnel, and methyl tert-butyl ether was added to the remaining liquid for low-temperature crystallization. The solution after crystallization was removed by centrifugation. The precipitates were washed with methyl tert-butyl ether, and dried in vacuum at 25-35°C to obtain a crude peptide of Ac-D-Cys-D-Ala-D-Arg-D-Arg-D-Arg-D-Ala-D-Arg-NH$_2$. LC-MS (ESI): m/z 930.3 [M+H]$^+$.

(2) The peptide fragment H$_2$N-D-Tyr-Aib-D-Phe-Gly-Gly-L-Cys-OH (polypeptide sequence shown in SEQ ID NO: 5) was prepared by synthesizing from the C-terminal to the N-terminal.

(2-1) Pretreatment of resin: 2-CTC resins were weighed out and swollen with DCM.

(2-2) Coupling S8 amino acid: Fmoc-L-Cys(Trt)-OH was weighed out, dissolved in DCM, and added into DIEA. The obtained mixture was added to a reaction test tube, and nitrogen was introduced and the mixture was stirred for 1-3 hours. Then a mixed solution of methanol and DIEA (DIEA: methanol (v/v) = 1:9) was added to block the reaction for 10-20 min. The solvent was drained and the resins were washed with DCM followed by DMF. Piperidine/DMF solution was added the resins for deprotection for 20-30 min, and then the reagent was drained. The resins were washed with DMF and the solvent was drained. The resins were then subjected to ninhydrin test. When the color of the resins was purplish-black, the next step was carried out.

(2-3) Fmoc-Gly-OH was weighed and linked by step (1-2) as S9 amino acid. Step (2-3) was then repeated to link S10 amino acid of Fmoc-Gly-OH.

(2-4) Fmoc-D-Phe-OH was weighed and linked by step (1-2) as S11 amino acid.

(2-5) Fmoc-Aib-OH was weighed and linked by step (1-2) as S12 amino acid.

(2-6) Fmoc-D-Tyr(t-Bu)-OH was weighed and linked as S13 amino acid.

(2-7) Resin washing was performed as described in step (1-8).

(2-8) Cleavage of peptide: 155 mL of TFA, 8 mL of TIS (triisopropyl silane), 4.12 mL of EDT (ethanedithiol), 2 mL of TA (thioanisole), 4.12 mL of water, and 2 mL of anisole were mixed evenly to prepare a lysis buffer. After the peptide fragment was cleaved, the buffer was removed by filtration under reduced pressure with a sand core funnel, and methyl tert-butyl ether was added to the remaining liquid for low-temperature crystallization. The solution after crystallization was removed by centrifugation. The precipitates were washed with methyl tert-butyl ether, and dried in vacuum at 25-35°C to obtain a crude peptide of $H_2N$-D-Tyr-Aib-D-Phe-Gly-Gly-L-Cys-OH. LC-MS (ESI): m/z 631.6 [M+H]$^+$.

After the peptide fragment was cleaved, the buffer was removed by filtration under reduced pressure with a sand core funnel, and methyl tert-butyl ether was added to the remaining liquid for low-temperature crystallization. The solution after crystallization was removed by centrifugation. The precipitates were washed with methyl tert-butyl ether, and dried in vacuum at 25-35°C to obtain the peptide segment

Linking: The crude peptide Ac-D-Cys-D-Ala-D-Arg-D-Arg-D-Arg-D-Ala-D-Arg-$NH_2$ prepared in step (1-9) of Example 1 and the crude peptide $H_2N$-D-Tyr -Aib-D-Phe-Gly-Gly-L-Cys-OH prepared in step (2-8) of Example 1 were weighed and dissolved in purified water, and then DMSO was added to react. After mixing well, the reaction system was stirred at 20-30°C. The completion of the reaction was detected by HPLC and the polypeptide (SEQ ID NO: 10) solution was obtained.

(4) Purification: The crude peptide solution obtained in step (3) of Example 1 was filtered through a 0.45 µm filter membrane, and then purified by preparative HPLC on a 20mm×150mm column filled with 10 µm C-18 silica gel. The detection wavelength was 220 nm. Mobile phase A was 0.1% TFA and mobile phase B was acetonitrile. After gradient elution, the fractions containing the target polypeptide product were collected with a purity of 95.8%. The collected fractions were combined, the solvent was removed under reduced pressure and the polypeptide compound was lyophilized. The final product obtained was identified by analytical RP-HPLC (retention time) and LC-MS. LC-MS (ESI): m/z 1558.5 [M+H]$^+$. By QE identification and analysis, the sequence of the obtained polypeptide was confirmed as SEQ ID NO: 10.

**Example 2: Preparation of Active Polypeptide (SEQ ID NO: 11)**

Preparation of peptide fragment H-D-Tyr-D-Arg-D-Phe-Gly-Gly-L-Cys-OH (polypeptide sequence shown in SEQ ID NO: 6): According to the preparation method of step (2) in Example 1, 2-CTC resins were used to link Fmoc-L-Cys(Trt)-OH, Fmoc-Gly-OH, Fmoc-Gly-OH, Fmoc-D-Phe-OH, Fmoc-D-Arg(pbf)-OH, Fmoc-D-Tyr(t-Bu)-OH in order to obtain a crude peptide of H-D-Tyr-D-Arg-D-Phe-Gly-Gly-L-Cys-OH. LC-MS (ESI): m/z 702.8 $[M+H]^+$.

(2) Linking: The crude peptide

Ac-D-Cys-D-Ala-D-Arg-D-Arg-D-Arg-D-Ala-D-Arg-NH$_2$ prepared in step (1) of Example 1 and the crude peptide H-D-Tyr-D-Arg-D-Phe-Gly-Gly-L-Cys-OH prepared in step (1) of Example 2 were weighed and dissolved in purified water, and then added into DMSO to react. The completion of reaction was detected by HPLC and the polypeptide (SEQ ID NO: 11) solution was obtained.

(3) Purification: The freeze-dried polypeptide compound was obtained using the method of step (4) of Example 1. The final product obtained was identified by analytical RP-HPLC (retention time) and LC-MS. LC-MS (ESI): m/z 1629.3 $[M+H]^+$. By QE identification and analysis, the sequence of the obtained polypeptide was confirmed as SEQ ID NO: 11.

**Example 3: Preparation of Active Polypeptide (SEQ ID NO: 7)**

```
Ac——D-Cys——D-Ala——D-Arg——D-Arg——D-Arg——D-Ala——D-Arg——NH₂
              |
              S——S
                  |
H₂N——L-Tyr——L-Cys——L-Phe——Gly——Gly——OH
```

(1) Preparation of peptide fragment H$_2$N-L-Tyr-L-Cys-L-Phe-Gly-Gly-OH (polypeptide sequence shown in SEQ ID NO: 2): According to the preparation method of step (2) in Example 1, 2-CTC resins were used to link Fmoc-Gly-OH, Fmoc-Gly-OH, Fmoc-L-Phe-OH, Fmoc-L-Cys(Trt)-OH, Fmoc-D-Tyr(t-Bu)-OH in order to obtain a crude peptide of peptide fragment H$_2$N-L-Tyr-L-Cys-L-Phe-Gly-Gly-OH. LC-MS (ESI): m/z 546.2 $[M+H]^+$.

(2) Linking: The crude peptide
Ac-D-Cys-D-Ala-D-Arg-D-Arg-D-Arg-D-Ala-D-Arg-NH$_2$ prepared in step (1) of Example 1 and the crude peptide H$_2$N-L-Tyr-L-Cys-L-Phe-Gly-Gly-OH prepared in step (1) of Example 3 were weighed and dissolved in purified water, and then added into DMSO to react. The completion of reaction was detected by HPLC, the polypeptide (SEQ ID NO: 7) solution was obtained.

(3) Purification: The freeze-dried polypeptide compound was obtained using the method of step (4) of Example 1. The final product obtained was identified by analytical RP-HPLC (retention time) and LC-MS. LC-MS (ESI): m/z 1473.2 $[M+H]^+$. By QE identification and analysis, the sequence of the obtained polypeptide was confirmed as SEQ ID NO: 7.

**Example 4: Preparation of Active Polypeptide (SEQ ID NO: 8)**

```
Ac——D-Cys——D-Ala——D-Arg——D-Arg——D-Arg——D-Ala——D-Arg——NH₂
              |
              S——S
                  |
H₂N——D-Tyr——L-Cys——D-Phe——Gly——Gly——OH
```

(1) Preparation of peptide fragment H$_2$N-D-Tyr-L-Cys-D-Phe-Gly-Gly-OH (polypeptide sequence shown in SEQ ID NO: 3): According to the preparation method of step (2) in Example 1, 2-CTC resins were used to link Fmoc-Gly-OH, Fmoc-Gly-OH, Fmoc-D-Phe-OH, Fmoc-L-Cys(Trt)-OH, Fmoc-D-Tyr(t-Bu)-OH in order to obtain a crude peptide of peptide fragment H$_2$N-D-Tyr-L-Cys-D-Phe-Gly-Gly-OH. LC-MS (ESI): m/z 546.2 $[M+H]^+$.

(2) Linking: The crude peptide
Ac-D-Cys-D-Ala-D-Arg-D-Arg-D-Arg-D-Ala-D-Arg-NH$_2$ prepared in step (1) of Example 1 and the crude peptide

$H_2N$-D-Tyr-L-Cys-D-Phe-Gly-Gly-OH (90 mg) prepared in step (1) of Example 4 were weighed and dissolved in purified water, and then added into DMSO to react. After mixing well, the reaction was stirred at 30-40°C for 10 hours. The completion of reaction was detected by HPLC, the polypeptide (SEQ ID NO: 8) solution was obtained.

(3) Purification: The freeze-dried polypeptide compound was obtained using the method of step (4) of Example 1. The final product obtained was identified by analytical RP-HPLC (retention time) and LC-MS. LC-MS (ESI): m/z 1473.2 [M+H]+. By QE identification and analysis, the sequence of the obtained polypeptide was confirmed as SEQ ID NO: 8.

**Example 5: Preparation of Active Polypeptide (SEQ ID NO: 9)**

```
Ac——D-Cys——D-Ala——D-Arg——D-Arg——D-Arg——D-Ala——D-Arg——NH2
          |
          S——S
                |
H2N——D-Tyr——D-Cys——D-Phe——Gly——Gly——OH
```

(1) Preparation of peptide fragment $H_2N$-D-Tyr-D-Cys-D-Phe-Gly-Gly-OH (polypeptide sequence shown in SEQ ID NO: 4): According to the preparation method of step (2) in Example 1, 2-CTC resins were used to link Fmoc-Gly-OH, Fmoc-Gly-OH, Fmoc-D-Phe-OH, Fmoc-D-Cys(Trt)-OH, Fmoc-D-Tyr(t-Bu)-OH in order to obtain a crude peptide of peptide fragment $H_2N$-D-Tyr-D-Cys-D-Phe-Gly-Gly-OH. LC-MS (ESI): m/z 546.2 [M+H]+.

(2) Linking: The crude peptide Ac-D-Cys-D-Ala-D-Arg-D-Arg-D-Arg-D-Ala-D-Arg-$NH_2$ prepared in step (1) of Example 1 and the crude peptide $H_2N$-D-Tyr-D-Cys-D-Phe-Gly-Gly-OH prepared in step (1) of Example 5 were weighed and dissolved in purified water, and then added into DMSO to react. The completion of reaction was detected by HPLC, the polypeptide (SEQ ID NO: 9) solution was obtained.

(3) Purification: The freeze-dried polypeptide compound was obtained using the method of step (4) of Example 1. The final product obtained was identified by analytical RP-HPLC (retention time) and LC-MS. LC-MS (ESI): m/z 1473.2 [M+H]+. By QE identification and analysis, the sequence of the obtained polypeptide was confirmed as SEQ ID NO: 9.

**Example 6**

According to the above preparation process, solid-phase synthesis was carried out to sequentially link amino acids to prepare the X peptide chain and the Y peptide chain, respectively. Finally, according to the linking method in Example 1, the X chain and the Y chain were connected by thiol condensation, and the following polypeptide compounds SEQ ID NOs: 19-22 were also prepared. The required amino acid raw materials could be purchased from the market.

(1) SEQ ID NO: 19
(2) SEQ ID NO: 20

```
Ac——D-Cys——D-Arg——D-Arg——D-Ala——D-Arg——D-Ala——D-Arg——NH2
          |
          S——S
                   \
H2N——D-Tyr——Aib——D-Phe——Gly——Gly——L-Cys——OH        ;
```

(3) SEQ ID NO: 21
(4) SEQ ID NO: 22

In addition to the above polypeptide compounds, the X chain moiety in the polypeptide compound might also be prepared as a cyclic peptide by linking the amino terminal and carboxyl terminal. First, the same or similar solid-phase synthesis method for SEQ ID NO: 5 in Example 1 was used to prepare the X chain with free carboxyl terminal and amino terminal. Then, at an appropriate pH, the X chain was reacted with $(Boc)_2O$ to selectively protect the amino group. The carboxyl terminal was prepared into p-nitrophenol ester by the active ester method, and the Boc protecting group of the amino acid was removed with TFA. 0.1M NaHCOs and 0.1M $Na_2CO_3$ were used as bases and dioxane as a solvent to carry out the reaction at room temperature to obtain cyclic peptide X with a yield of about 20%.

Finally, according to the peptide chain linking method in Example 1, the cyclic peptide X and Y chain were connected by disulfide bond to obtain the following polypeptide compounds: SEQ ID NOs: 23-27.

(5) SEQ ID NO: 23

Ac——D-Cys——D-Ala——D-Arg——D-Arg——D-Arg——D-Ala——D-Arg——$NH_2$

S——S

Cyclo ( D-Tyr——Aib——D-Phe——Gly——Gly——L-Cys) ;

(6) SEQ ID NO: 24
(7) SEQ ID NO: 25
(8) SEQ ID NO: 26
(9) SEQ ID NO: 27

Ac——D-Cys——D-Ala——D-Arg——D-Arg——D-Arg——D-Ala——D-Arg——$NH_2$

S——S

Cyclo ( D-Cys——Gly——Gly——D-Phe——Gly——D-Tyr) .

**Effect Examples**

**(1) Effects of single intravenous injection on serum Ca, P and PTH in normal rats**

Test method: SPF grade SD rats, weighing 250-280 g, were randomly divided into 7 groups, 6 administration groups and 1 blank control group (Vehicle = normal saline). The test samples were prepared into a solution with normal saline for administration. The test compound was administered by a single tail vein injection (1 mL/kg) at a dose of 0.5 mg/kg, 1.5 mg/kg and 4.5 mg/kg, respectively. Serum calcium (mmol/L) and phosphorus levels (mmol/L) were measured at 0 hour and at 4, 8 and 16 hours after administration.

Data processing: The concentrations of PTH, calcium and phosphorus in each group and at each time point were statistically analyzed using the graphpad6.0 software.

Experimental results: the experimental results of changes in blood calcium levels of rats in each group are shown in Table 1, and the experimental results of changes in blood phosphorus levels of rats are shown in Table 2.

Table 1: Changes of blood calcium levels after a single intravenous bolus injection (mmol/L)

| Dosing group | Dosage | 0 h | 4 h | 8 h | 16 h |
|---|---|---|---|---|---|
| Normal saline | -- | 2.51±0.02 | 2.29±0.02 | 2.25±0.01 | 2.37±0.01 |
| Administration group/Example 1 | 0.5 | 2.46±0.02 | 2.04±0.04 | 2.18±0.06 | 2.26±0.02 |
| | 1.5 | 2.47±0.02 | 1.91±0.02 | 1.96±0.02 | 2.19±0.02 |
| | 4.5 | 2.46±0.02 | 1.60±0.03 | 1.46±0.04 | 1.53±0.05 |
| Administration group/Example 2 | 0.5 | 2.42±0.02 | 2.05±0.04 | 2.20±0.02 | 2.27±0.01 |
| | 1.5 | 2.41±0.06 | 2.02±0.06 | 1.84±0.04 | 2.10±0.03 |
| | 4.5 | 2.45±0.02 | 1.79±0.03 | 1.53±0.03 | 1.64±0.17 |

It can be seen from Table 1 that the polypeptides prepared in Examples 1 and 2 of the present disclosure was administered to normal rats by a single tail vein bolus injection, and samples were taken at 4 h, 8 h and 16 h after administration. Compared with the concentration before administration (0 h), the blood calcium levels of rats in each administration group decreased significantly at 0, 4, 8, and 16 h (P≤0.01).

Table 2: Changes of blood phosphorus levels after a single intravenous bolus injection (mmol/L)

| Dosing group | Dosage | 0 | 4 h | 8 h | 16 h |
|---|---|---|---|---|---|
| Normal saline | -- | 2.69±0.07 | 2.40±0.08 | 2.40±0.1 | 2.42±0.08 |
| Administration group/Example 1 | 0.5 | 2.72±0.1 | 2.74±0.06 | 2.51±0.05 | 2.33±0.06 |
| | 1.5 | 2.64±0.06 | 2.96±0.09 | 2.82±0.05 | 2.59±0.12 |
| | 4.5 | 2.61±0.09 | 2.89±0.05 | 3.53±0.08 | 2.75±0.07 |
| Administration group/Example 2 | 0.5 | 2.70±0.06 | 2.78±0.14 | 2.67±0.06 | 2.49±0.08 |
| | 1.5 | 2.65±0.08 | 3.04±0.08 | 3.05±0.08 | 2.47±0.06 |
| | 4.5 | 2.63±0.02 | 3.31±0.08 | 3.67±0.1 | 2.49±0.06 |

It can be seen from Table 2 that the both polypeptides prepared in Examples 1 and 2 of the present disclosure administered to normal rats by a single tail vein bolus injection had the effect of increasing the blood phosphorus concentration. Compared with the concentration before administration (0 h), the serum phosphorus level in the low-dose group (0.5 mg/kg and 1.5 mg/kg) increased during 0-4 h, and reached the highest at 4 h; and the concentration in the high-dose group (4.5 mg/kg) increased during 0-8 h, and reached the highest at 8 h.

**(2) Determination of calcimimetic activity of test compounds on calcium-sensing receptor (CASR)**

In the CaSR activation experiment, the Homogeneous Time-Resolved Fluorescence (HTRF) IP-1 (myoInositol 1 phosphate) competition method was used to detect the effect of the polypeptide of Example 1 on CaSR. The experimental results show that the polypeptide of Example 1 was a CaSR agonist, showing significant agonistic activity on HEK293-CaSR cells at a $Ca^{2+}$ concentration of 1.2 mM, with an $EC_{50}$ of 84.68 $\mu$M; in the absence of $Ca^{2+}$, the agonist activity on HEK293-CaSR cells was weak, with an $EC_{50}$ of 979.60 $\mu$M. In conclusion, the polypeptide of Example 1 had the effect of activating CaSR, and the activation process required the synergistic function of $Ca^{2+}$.

**(3) Effects of test compounds on plasma PTH in 5/6 nephrectomy rats**

Male SD rats were divided into a sham operation group and a 5/6 nephrectomy group (5/6Nx). Rats in the nephrectomy group underwent 5/6 nephrectomy, and were fed with high-phosphorus diet for two weeks after the operation to accelerate the induction of the secondary hyperparathyroidism rat model. After being fed with high-phosphorus diet for 3 weeks, the model rats were randomly divided into 6 groups, a 5/6Nx group (Vehicle), a positive control drug etelcalcetide group (3.0 mg/kg) and 4 groups of the compound of Example 1 (0.5, 1.0, 2.0, 4.0 mg/kg). The rats were administered subcutaneously three times a week for 6 consecutive weeks. Before the first administration, after the first administration, and 4 hours after administration in the 2nd, 4th, and 6th week, blood samples were collected from rats, and the plasma and serum were separated for the detection of the content of PTH in the plasma at each time point.

Table 3 Percentage change of PTH in plasma 4h after administration of polypeptide of Example 1

| Group | Dosage (mg/kg) | PTH change (%) | | | |
|---|---|---|---|---|---|
| | | First administration | 2 weeks | 4 weeks | 6 weeks |
| Vehicle | -- | 9.97±7.74 | 53.46±24.40 | 5.86±8.17 | 20.13±8.95 |
| Positive control | 3.0 | -92.03±0.67 | -95.57±0.28 | -93.81±0.59 | -92.63±0.77 |
| Example 1 | 0.5 | -75.68±4.71 | -75.30±7.87 | -62.15±7.90 | -62.51±8.63 |
| | 1.0 | -90.25±2.40 | -93.54±1.30 | -88.78±3.38 | -75.71±7.55 |
| | 2.0 | -95.13±0.70 | -94.39±0.89 | -94.36±1.01 | -94.04±1.16 |
| | 4.0 | -94.08±0.90 | -93.87±0.86 | -94.96±0.66 | -94.79±0.50 |

At different time points of the first administration and the 2nd, 4th, and 6th weeks of continuous administration, compared with the 5/6 nephrectomy group (Vehicle), the PTH content in the plasma of the rats in Example 1 polypeptide administration group (1.0, 2.0, 4.0 mg/kg) and the positive drug etelcalcetide group (3.0 mg/kg) was significantly reduced 4 hours after administration, and the reduction of plasma PTH was as high as 90% or more.

**(4) Validation of the osteogenic growth peptide-like activity of the polypeptide prepared in Example 1**

The CyQUANT cell proliferation detection kit was used to determine the *in vitro* proliferation effect of the polypeptide of Example 1 on osteoblast MC3T3-E1 Subclone 4 and fibroblast NIH/3T3 cells. Osteogenic growth peptide (OGP) was used as a positive control in the experiment. The polypeptide of Example 1 and OGP were prepared to the following concentrations: $10^{-8}$ mol/L, $10^{-9}$ mol/L, $10^{-10}$ mol/L, $10^{-11}$ mol/L, $10^{-12}$ mol/L, $10^{-13}$ mol/L, $10^{-14}$ mol/L, $10^{-15}$ mol/L and $10^{-16}$ mol/L. 100 $\mu$L of medium was discarded from each well of the cell plate to be tested, and 100 $\mu$L of 2 $\times$ CyQuant detection reagent was added to each well. The plate was then placed in a 37°C, 5% $CO_2$ incubator for 60 min. Fluorescence values were read in bottom-reading mode with excitation and emission wavelengths of 480/535 nm.

$$\% \text{ relative to Control} = (\text{reading value of compound well - reading value of blank control well}) / (\text{reading value of base control well - reading value of blank control well}) \times 100$$

The experimental data in FIG. 1 shows that the polypeptide of Example 1 significantly stimulated the proliferation of osteoblast MC3T3-E1 Subclone 4 cells at a concentration of $10^{-8}$ to $10^{-13}$ M, which was more pronounced than that of OGP at a concentration of $10^{-8}$ to $10^{-16}$ M. The polypeptide of Example 1 significantly stimulates the proliferation of fibroblast NIH3T3 cells at $10^{-8}$ M, with an effective dose comparable to that of OGP at $10^{-8}$ M.

**(5) Effects of the polypeptide of Example 1 on cortical osteoporosis in adenine-gavage rats**

According to the Yokozawz method, SD rats were given adenine gavage to establish an animal model of chronic renal failure, and fed with a high-phosphorus diet to induce a model of secondary hyperparathyroidism. Rats were fed until the end of the experiment, and model rats were screened according to the content of PTH in plasma. According to the PTH content and body weight, the model rats were randomly divided into 5 groups: a normal saline group, an etelcalcetide (3.0 mg/kg, dissolved in normal saline) group, 3 groups of Example 1 (1.0 mg/kg, 2.0 mg /kg and 4.0 mg/kg, dissolved in normal saline), and subcutaneously administered three times a week for six consecutive weeks at 1.0 ml/kg per rat. Six weeks later, the left femur of the rat was taken, the muscle and connective tissue were removed, and the femur was placed in a 4% formalin solution. Micro CT scanning was performed 24 hours later, and data was subjected analysis.

Table 4 Effects on cortical bone porosity of rats in adenine gavage group after administration of polypeptide of Example 1 (Mean±SEM)

| Group | 0.5% MC | Model group | Etelcalcetide | Example 1 | | |
|---|---|---|---|---|---|---|
| Dosage (mg/kg) | -- | -- | 3.0 | 1.0 | 2.0 | 4.0 |
| Cortical bone porosity (%) | 0.06±0.02 | 11.28±3.83 | 4.13±2.27 | 6.25±2.22 | 4.11±1.54 | 0.66±0.22 |

Rats of adenine-induced SHPT rat model developed cortical osteoporosis, and the cortical bone porosity was significantly increased. Compared with rats in the adenine model group (Vehicle), the cortical bone porosity of the rats in the Example 1 administration group was significantly reduced (see Table 4). Continuous administration of the polypeptide of Example 1 for 6 weeks significantly reduced the cortical bone porosity and increase the cortical bone density of adenine-induced SHPT rats, with an effect better than that of etelcalcetide.

The above content is a further detailed description of the present disclosure in combination with specific preferred embodiments, and it shall not be considered that the specific implementation of the present disclosure is limited to these descriptions. For those of ordinary skill in the technical field of the present disclosure, some simple deductions or substitutions may be made without departing from the concept of the present disclosure, which should be regarded as belonging to the protection scope of the present disclosure.

**Claims**

1. A polypeptide compound having a structure represented by the following formula (Ia) or formula (Ib), or a pharmaceutically acceptable salt thereof:

Y-ID-X          formula (Ia),

or

X-ID-Y formula          (Ib);

wherein,

Y is a calcium-sensing receptor agonist,
ID is an intramolecular disulfide bond or a linker connecting X and Y, and
X is an osteogenic growth peptide-like peptide or a stimulator of bone marrow mesenchymal stem cells.

2. The polypeptide compound according to claim 1, wherein Y is a peptide chain comprising an amino acid sequence set forth in formula (IIa) or formula (IIb):

$Xaa_7$-$Xaa_8$-$Xaa_9$-$Xaa_{10}$-$Xaa_{11}$-$Xaa_{12}$-$Xaa_{13}$          formula (IIa),

$Xaa_{13}$-$Xaa_{12}$-$Xaa_{11}$-$Xaa_{10}$-$Xaa_9$-$Xaa_8$-$Xaa_7$          formula (IIb);

wherein,

$Xaa_7$ is selected from the group consisting of cysteine, homocysteine and S-methylcysteine;
$Xaa_8$ is selected from the group consisting of alanine and arginine;
$Xaa_9$ is selected from the group consisting of arginine, lysine and histidine;
$Xaa_{10}$ is selected from the group consisting of arginine, alanine, lysine and histidine;
$Xaa_n$ is selected from the group consisting of arginine, lysine and histidine;
$Xaa_{12}$ is alanine;
$Xaa_{13}$ is selected from the group consisting of arginine, lysine and histidine;
the amino terminal of the peptide chain Y is free or chemically modified, and the carboxyl terminal of the peptide chain Y is free or chemically modified;
$Xaa_8$ is preferably selected from the group consisting of D-alanine and D-arginine;
$Xaa_9$ is preferably selected from the group consisting of D-arginine, D-lysine and D-histidine;
$Xaa_{10}$ is preferably selected from the group consisting of D-arginine, D-alanine, D-lysine and D-histidine;
$Xaa_n$ is preferably selected from the group consisting of D-arginine, D-lysine and D-histidine;
$Xaa_{12}$ is preferably D-alanine; and
$Xaa_{13}$ is preferably selected from the group consisting of D-arginine, D-lysine and D-histidine.

3. The polypeptide compound according to claim 1 or 2, wherein X is a peptide chain comprising an amino acid sequence set forth in formula (IIIa) or formula (IIIb):

$Xaa_1$-$Xaa_2$-$Xaa_3$-$Xaa_4$-$Xaa_5$-$Xaa_6$          (IIIa),

$Xaa_6$-$Xaa_5$-$Xaa_4$-$Xaa_3$-$Xaa_2$-$Xaa_1$          (IIIb);

wherein,

$Xaa_1$ is selected from the group consisting of tyrosine, homotyrosine and 3-chlorotyrosine;
$Xaa_2$ is selected from the group consisting of cysteine, homocysteine, S-methylcysteine, 2-aminoisobutyric acid, arginine, proline, glycine, homoarginine, lysine, cyclohexylalanine, sarcosine, norleucine and histidine;
$Xaa_3$ is selected from the group consisting of phenylalanine, tryptophan and tyrosine;
$Xaa_4$ is glycine, $Xaa_5$ is glycine, or $Xaa_4$-$Xaa_5$ is replaced by an amino-substituted $C_{1\text{-}20}$ alkyl monocarboxylic acid;
when $Xaa_2$ is selected from the group consisting of cysteine and homocysteine, $Xaa_6$ is absent;

when $Xaa_2$ is selected from the group consisting of S-methylcysteine, 2-aminoisobutyric acid, arginine, proline, glycine, homoarginine, lysine, cyclohexylalanine, sarcosine, norleucine and histidine, $Xaa_6$ is selected from the group consisting of cysteine and homocysteine;

the amino terminal of the peptide chain X is free or chemically modified, and the carboxyl terminal of the peptide chain X is free or chemically modified;

$Xaa_1$ is preferably selected from the group consisting of L-tyrosine, D-tyrosine, D-homotyrosine and 3-chloro-D-tyrosine;

$Xaa_2$ is preferably selected from the group consisting of L-cysteine, D-cysteine, D-homocysteine, L-homocysteine, S-methyl-D-cysteine, 2-aminoisobutyl acid, D-arginine, L-arginine, L-proline, D-proline, glycine, D-histidine and L-cyclohexylalanine;

$Xaa_3$ is preferably selected from the group consisting of L-phenylalanine, D-phenylalanine, D-tryptophan, L-tryptophan, D-tyrosine and L-tyrosine;

$Xaa_4$ is preferably glycine, $Xaa_5$ is glycine, or $Xaa_4$-$Xaa_5$ is replaced by an amino-substituted $C_{1-20}$ alkyl mono-carboxylic acid;

preferably when $Xaa_2$ is selected from the group consisting of L-cysteine, D-cysteine, D-homocysteine and L-homocysteine, $Xaa_6$ is absent;

preferably when $Xaa_2$ is selected from the group consisting of S-methyl-D-cysteine, 2-aminoisobutyric acid, D-arginine, L-arginine, L-proline, D-proline, glycine, D-histidine and L-cyclohexylalanine, $Xaa_6$ is selected from the group consisting of L-cysteine, D-cysteine, D-homocysteine and L-homocysteine.

4. The polypeptide compound according to claim 1 or 2, X is a cyclic peptide having a structure represented by formula (IIIc) or (IIId):

Cyclo ($Xaa_1$-$Xaa_2$-$Xaa_3$-$Xaa_4$-$Xaa_5$-$Xaa_6$)        (IIIc),

Cyclo ($Xaa_6$-$Xaa_5$-$Xaa_4$-$Xaa_3$-$Xaa_2$-$Xaa_1$)        (IIId);

wherein,

$Xaa_1$ is selected from the group consisting of tyrosine, homotyrosine and 3-chlorotyrosine;

$Xaa_2$ is selected from the group consisting of cysteine, homocysteine, S-methylcysteine, 2-aminoisobutyric acid, arginine, proline, glycine, homoarginine, lysine, cyclohexylalanine, sarcosine, norleucine and histidine;

$Xaa_3$ is selected from the group consisting of phenylalanine, tryptophan and tyrosine;

$Xaa_4$ is glycine;

$Xaa_5$ is glycine;

when $Xaa_2$ is selected from the group consisting of cysteine and homocysteine, $Xaa_6$ is absent, and the amino group of $Xaa_1$ forms a peptide bond with the carboxyl group of $Xaa_5$, or the amino group of $Xaa_5$ forms a peptide bond with the carboxyl group of $Xaa_1$;

when $Xaa_2$ is selected from the group consisting of S-methylcysteine, 2-aminoisobutyric acid, arginine, proline, glycine, homoarginine, lysine, cyclohexylalanine, sarcosine, norleucine and histidine, $Xaa_6$ is selected from the group consisting of cysteine and homocysteine, and the amino group of $Xaa_1$ forms a peptide bond with the carboxyl group of $Xaa_6$, or the amino group of $Xaa_6$ forms a peptide bond with the carboxyl group of $Xaa_1$;

$Xaa_1$ is preferably selected from the group consisting of L-tyrosine, D-tyrosine, D-homotyrosine and 3-chloro-D-tyrosine;

$Xaa_2$ is preferably selected from the group consisting of L-cysteine, D-cysteine, D-homocysteine, L-homocysteine, S-methyl-D-cysteine, 2-aminoisobutyl acid, D-arginine, L-arginine, L-proline, D-proline, glycine, D-histidine and L-cyclohexylalanine;

$Xaa_3$ is preferably selected from the group consisting of L-phenylalanine, D-phenylalanine, D-tryptophan, L-tryptophan, D-tyrosine and L-tyrosine;

$Xaa_4$ is preferably glycine, $Xaa_5$ is glycine, or $Xaa_4$-$Xaa_5$ is replaced by an amino-substituted $C_{1-20}$ alkyl mono-carboxylic acid;

preferably when $Xaa_2$ is selected from the group consisting of L-cysteine, D-cysteine, D-homocysteine and L-homocysteine, $Xaa_6$ is absent;

preferably when $Xaa_2$ is selected from the group consisting of S-methyl-D-cysteine, 2-aminoisobutyric acid, D-arginine, L-arginine, L-proline, D-proline, glycine, D-histidine and L-cyclohexylalanine, $Xaa_6$ is selected from the group consisting of L-cysteine, D-cysteine, D-homocysteine and L-homocysteine.

5. The polypeptide compound according to any one of claims 1-4, wherein the chemical modification of the amino terminal of X or Y is selected from the group consisting of acylation, sulfonylation, alkylation and PEGylation modification; and the chemical modification of the carboxyl terminal of X or Y is selected from the group consisting of amidation, sulfonylation and PEGylation.

6. The polypeptide compound according to claim 5, wherein the chemical modification of the amino terminal is selected from the group consisting of acetylation, benzoylation, sulfonylation or alkylation, and the alkylation of the amino terminal is $C_{1-6}$ alkylation or aralkylation; and the chemical modification of the carboxyl terminal is that the OH of carboxyl group is substituted by $NH_2$ or sulfonamide.

7. The polypeptide compound according to any one of claims 1-6, wherein ID is an intramolecular disulfide bond or a linker formed between X and Y;

   wherein the intramolecular disulfide bond is formed between cysteine, homocysteine or S-methylcysteine in X and cysteine, homocysteine or S-methylcysteine in Y;
   the linker is selected from the group consisting of an amino-substituted $C_{1-8}$ alkyl acid, a polyethylene glycol polymer chain and a peptide fragment consisting of 1-10 amino acids, and the amino acid in the peptide fragment is selected from the group consisting of proline, arginine, alanine, threonine, glutamic acid, aspartic acid, lysine, glutamine, asparagine and glycine;
   or the linker is selected from the group consisting of:

   (1) (Gly-Ser)$_n$; where n is 0, 1, 2 or 3;
   (2) Gly-Ser-Gly;
   (3) Ser-Gly-Gly-Ser-Gly-Gly-Ser;
   (4) 4-aminobutyric acid or 6-aminocaproic acid; and
   (5) (PEG)$_m$, where m is 1, 2, 3, 4, or 5.

8. The polypeptide compound according to claim 1 or 2, wherein X is a peptide chain selected from the group consisting of SEQ ID NOs: 2-6 and SEQ ID NOs: 12-18, wherein

   (1) SEQ ID NO: 2
   $H_2N$-L-Tyr-L-Cys-L-Phe-Gly-Gly-OH;
   (2) SEQ ID NO: 3
   $H_2N$-D-Tyr-L-Cys-D-Phe-Gly-Gly-OH;
   (3) SEQ ID NO: 4
   $H_2N$-D-Tyr-D-Cys-D-Phe-Gly-Gly-OH;
   (4) SEQ ID NO: 5
   $H_2N$-D-Tyr-Aib-D-Phe-Gly-Gly-L-Cys-OH;
   (5) SEQ ID NO: 6
   $H_2N$-D-Tyr-D-Arg-D-Phe-Gly-Gly-L-Cys-OH;
   (6) SEQ ID NO: 12
   $H_2N$-D-Tyr-D-His-D-Tyr-Gly-Gly-L-Cys-OH;
   (7) SEQ ID NO: 13
   $H_2N$-D-Tyr-D-Pro-D-Phe-Gly-Gly-L-Cys-OH;
   (8) SEQ ID NO: 14
   Cyclo (D-Tyr-Aib-D-Phe-Gly-Gly-L-Cys);
   (9) SEQ ID NO: 15
   Cyclo (D-Tyr-D-Arg-D-Phe-Gly-Gly-L-Cys);
   (10) SEQ ID NO: 16
   Cyclo (L-Tyr-Gly-L-Phe-Gly-Gly-L-Cys);
   (11) SEQ ID NO: 17
   Cyclo (D-Tyr-Gly-D-Phe-Gly-Gly-D-Cys); and
   (12) SEQ ID NO: 18
   Cyclo (D-Cys-Gly-Gly-D-Phe-Gly-D-Tyr).

9. The polypeptide compound according to claim 1, which is selected from the group consisting of SEQ ID NOs: 7-11 and SEQ ID NOs: 19-27, or a pharmaceutically acceptable salt thereof, wherein

   (1) SEQ ID NO: 7

```
Ac——D-Cys——D-Ala——D-Arg——D-Arg——D-Arg——D-Ala——D-Arg——NH₂
        |
        S——S
              |
H₂N——L-Tyr——L-Cys——L-Phe——Gly——Gly——OH
```
,

(2) SEQ ID NO: 8

```
Ac——D-Cys——D-Ala——D-Arg——D-Arg——D-Arg——D-Ala——D-Arg——NH₂
        |
        S——S
              |
H₂N——D-Tyr——L-Cys——D-Phe——Gly——Gly——OH
```
,

(3) SEQ ID NO: 9

```
Ac——D-Cys——D-Ala——D-Arg——D-Arg——D-Arg——D-Ala——D-Arg——NH₂
        |
        S——S
              |
H₂N——D-Tyr——D-Cys——D-Phe——Gly——Gly——OH
```
,

(4) SEQ ID NO: 10

```
Ac——D-Cys——D-Ala——D-Arg——D-Arg——D-Arg——D-Ala——D-Arg——NH₂
          \
           S——S
                \
H₂N——D-Tyr——Aib——D-Phe——Gly——Gly——L-Cys——OH
```
,

(5) SEQ ID NO: 11

```
Ac——D-Cys——D-Ala——D-Arg——D-Arg——D-Arg——D-Ala——D-Arg——NH₂
          \
           S——S
                \
H₂N——D-Tyr——D-Arg——D-Phe——Gly——Gly——L-Cys——OH
```
,

(6) SEQ ID NO: 19

```
Ac——D-Cys——D-Arg——D-Lys——D-Lys——D-Arg——D-Ala——D-Arg——NH₂
        |
        S——S
              |
H₂N——L-Tyr——L-Cys——L-Phe——Gly——Gly——OH
```
,

(7) SEQ ID NO: 20

```
Ac——D-Cys——D-Arg——D-Arg——D-Ala——D-Arg——D-Ala——D-Arg——NH₂
            |
            S——S
                  \
H₂N——D-Tyr——Aib——D-Phe——Gly——Gly——L-Cys——OH
```
                                                                        ,

(8) SEQ ID NO: 21

```
Ac——D-Cys——D-Ala——D-Arg——D-Arg——D-Arg——D-Ala——D-Arg——NH₂
            |
            S——S
                  \
H₂N——D-Tyr——D-His——D-Tyr——Gly——Gly——L-Cys——OH
```
                                                                        .

(9) SEQ ID NO: 22

```
Ac——D-Cys——D-Ala——D-Arg——D-Arg——D-Arg——D-Ala——D-Arg——NH₂
            |
            S——S
                  \
H₂N——D-Tyr——D-Pro——D-Phe——Gly——Gly——L-Cys——OH
```
                                                                        ,

(10) SEQ ID NO: 23

```
Ac——D-Cys——D-Ala——D-Arg——D-Arg——D-Arg——D-Ala——D-Arg——NH₂
            |
            S——S
                  \
Cyclo (D-Tyr——Aib——D-Phe——Gly——Gly——L-Cys)
```
                                                                        .

(11) SEQ ID NO: 24

```
Ac——D-Cys——D-Ala——D-Arg——D-Arg——D-Arg——D-Ala——D-Arg——NH₂
            |
            S——S
                  \
Cyclo (D-Tyr——D-Arg——D-Phe——Gly——Gly——L-Cys)
```
                                                                        ,

(12) SEQ ID NO: 25

```
Ac——D-Cys——D-Ala——D-Arg——D-Arg——D-Arg——D-Ala——D-Arg——NH₂
            |
            S——S
                  \
Cyclo (L-Tyr——Gly——L-Phe——Gly——Gly——L-Cys)
```
                                                                        ,

(13) SEQ ID NO: 26

Ac——D-Cys——D-Ala——D-Arg——D-Arg——D-Arg——D-Ala——D-Arg——NH$_2$

S——S

Cyclo ( D-Tyr——Gly——D-Phe——Gly——Gly——D-Cys)

, and

(14) SEQ ID NO: 27

Ac——D-Cys——D-Ala——D-Arg——D-Arg——D-Arg——D-Ala——D-Arg——NH$_2$

S——S

Cyclo ( D-Cys——Gly——Gly——D-Phe——Gly——D-Tyr)

.

10. Use of the polypeptide compound according to any one of claims 1-9 in the manufacture of a medicament exerting a calcium-sensing receptor agonist effect and/or an osteogenic growth peptide-like effect.

11. Use of the polypeptide compound according to any one of claims 1-9 in the manufacture of a medicament for preventing and/or treating a disease selected from the group consisting of an elevated parathyroid hormone disease, malignant hypercalcemia, metastasis bone disease, Paget's disease, osteoarthritis, rheumatoid arthritis, osteomalacia, chondro-calcinosis articularis, achondroplasia, osteochondritis, cystic osteogenesis imperfecta, congenital hypophosphatasia, fibromatous lesion, fibrous dysplasia, multiple myeloma, osteolytic bone disease, periprosthetic osteolysis, periodontal disease, osteoporosis, abnormal bone turnover, high turnover bone disease, chronic kidney disease-mineral, bone disorder (CKD-MBD) and a combination thereof; wherein the elevated parathyroid hormone disease is preferably primary hyperparathyroidism, secondary hyperparathyroidism, tertiary hyperparathyroidism, and the secondary hyperparathyroidism is preferably elevated parathyroid hormone caused by chronic kidney disease hemodialysis and/or peritoneal dialysis.

FIG. 1

## INTERNATIONAL SEARCH REPORT

| | International application No. |
| --- | --- |
| | **PCT/CN2021/088266** |

| A. | CLASSIFICATION OF SUBJECT MATTER |
| --- | --- |

C07K 7/06(2006.01)i; C07K 14/61(2006.01)i; A61K 38/02(2006.01)i; A61P 19/00(2006.01)i

According to International Patent Classification (IPC) or to both national classification and IPC

| B. | FIELDS SEARCHED |
| --- | --- |

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS, DWPI, CNTXT, WOTXT, USTXT, EPTXT, GBTXT, CATXT, SGTXT, ATTXT, LEXTXT, CHTXT, 万方数据库, CNKI, ISI Web of Science, PubMed, BAIDU AND SEARCH TERM, STN AND SEARCH TERM, NCBI, EMBL, China Patents Biological Sequence Search System: calcium, evocalcet, 拟钙剂, etelcalcetide, 钙敏感受体激动剂, OGPBP, cinacalcet, fusion protein, 西那卡赛, fusion, 钙敏感受体激活剂, CaSR, 融合, tecalcet, OGP, linker, 谷胱甘肽, 骨生长肽受体激活剂, 成骨生长肽, AMG 416, 维拉卡肽, NPS R-568, 连接, NPS R-467, 融合肽, 骨髓间充质干细胞刺激剂, 融合, 骨生长样肽, 骨髓基质细胞刺激剂, 盐酸依特卡肽, velcalcetide, parsabiv, calcimimetic, 钙敏感受体激活剂, osteogenic growth peptide, GSK3004774, 甲状腺旁激素, for SEQ ID NO: 7-11, SEQ ID NO: search for 19-27.

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 102548617 A (ACCELERON PHARMA, INC.) 04 July 2012 (2012-07-04) claims 1-42, description paragraphs 15-264 | 1-11 |
| A | CN 102573921 A (NEKTAR THERAPEUTICS) 11 July 2012 (2012-07-11) claims 1-21, description embodiments 1-13 | 1-11 |
| A | CN 102711789 A (KAI PHARMACEUTICALS INC.) 03 October 2012 (2012-10-03) claims 1-19, description paragraphs 18-345 | 1-11 |
| A | CN 109734778 A (SUZHOU UNIVERSITY OF SCIENCE AND TECHNOLOGY) 10 May 2019 (2019-05-10) description abstract, claim 1, description paragraphs 2-39 | 1-11 |
| A | CN 110054662 A (CHENGDU SHENGNUO BIOPHARM CO., LTD.) 26 July 2019 (2019-07-26) description abstract, claim 1, description paragraphs 2-35 | 1-11 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **05 July 2021** | **14 July 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2021/088266** |

## C.    DOCUMENTS CONSIDERED TO BE RELEVANT

| Category* | Citation of document, with indication, where appropriate, of the relevant passages | Relevant to claim No. |
|---|---|---|
| A | WO 2018152542 A1 (THE BOARD OF TRUSTEES OF THE LELAND STANFORD JUNIOR UNIVERSITY) 23 August 2018 (2018-08-23)<br>claims 1-6, description paragraphs 5-332 | 1-11 |
| A | WO 2005100571 A1 (NOVO NORDISK A/S et al.) 27 October 2005 (2005-10-27)<br>claims 1-8, description page 38 line 15- page 43 line 21 | 1-11 |
| A | US 2004126357 A1 (GENITRIX LLC) 01 July 2004 (2004-07-01)<br>description abstract, claims 1-11, description paragraphs 4-385 | 1-11 |
| A | 宋桉 等 (SONG, An et al.). "西那卡塞治疗原发性甲状旁腺功能亢进症 (Effects of Cinacalcet on Primary Hyperparathyroidism)"<br>中华骨质疏松和骨矿盐疾病杂志 (*Chinese Journal of Osteoporosis and Bone Mineral Research*), Vol. 10, No. 4, 10 July 2017 (2017-07-10),<br>abstract, and p. 394, left-hand column, paragraph 2 to p. 398, right-hand column, paragraph 4 | 1-11 |
| A | 燕宁 (YAN, Ning). "西那卡塞的临床应用以及研究进展 (non-official translation: Cinacalcet Clinical Application and Research Progress on Cinacalcet)"<br>中国血液净化 (*Chinese Journal of Blood Purification*),<br>Vol. 11, No. 8, 12 August 2017 (2017-08-12),<br>page 460, left-hand column, paragraph 2 to page 462, right-hand column, paragraph 1 | 1-11 |
| A | Liviawati Wu et al. "Drug disposition model of radiolabeled etelcalcetide in patients with chronic kidney disease and secondary hyperparathyroidism on hemodialysis"<br>*J Pharmacokinet Pharmacodyn*, No. 44, 06 January 2017 (2017-01-06),<br>abstract, page 47, right-hand column, paragraph 4 to page 52, right-hand column, paragraph 2, and figure 1 | 1-11 |
| A | Kevin J. Martin. "Velcalcetide (AMG 416), a novel peptide agonist of the calcium-sensing receptor, reduces serum parathyroid hormone and FGF23 levels in healthy male subjects"<br>*Nephrol Dial Transplant*, Vol. 29, No. 2, 13 November 2013 (2013-11-13),<br>abstract, p. 385, right-hand column, paragraph 3 to p. 391, left-hand column, paragraph 4 | 1-11 |

Form PCT/ISA/210 (second sheet) (January 2015)

**INTERNATIONAL SEARCH REPORT**

| International application No. |
|---|
| **PCT/CN2021/088266** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

        ☑ in the form of an Annex C/ST.25 text file.

        ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/CN2021/088266**

| Patent document cited in search report | | | Publication date (day/month/year) | Patent family member(s) | | | Publication date (day/month/year) |
|---|---|---|---|---|---|---|---|
| CN | 102548617 | A | 04 July 2012 | EP | 3702001 | A1 | 02 September 2020 |
| | | | | US | 2010266612 | A1 | 21 October 2010 |
| | | | | US | 8945877 | B2 | 03 February 2015 |
| | | | | EP | 3384964 | A1 | 10 October 2018 |
| | | | | CN | 107970445 | A | 01 May 2018 |
| | | | | EP | 3058986 | B1 | 09 May 2018 |
| | | | | AU | 2018201132 | B2 | 26 March 2020 |
| | | | | EP | 2414043 | B1 | 13 January 2016 |
| | | | | US | 9914762 | B2 | 13 March 2018 |
| | | | | JP | 5755635 | B2 | 29 July 2015 |
| | | | | AU | 2016204979 | A1 | 04 August 2016 |
| | | | | US | 2015266941 | A1 | 24 September 2015 |
| | | | | CA | 2757095 | C | 14 April 2020 |
| | | | | AU | 2010232693 | A1 | 03 November 2011 |
| | | | | EP | 3384964 | B1 | 25 March 2020 |
| | | | | WO | 2010114860 | A1 | 07 October 2010 |
| | | | | KR | 20170134788 | A | 06 December 2017 |
| | | | | JP | 2019146568 | A | 05 September 2019 |
| | | | | CA | 2757095 | A1 | 07 October 2010 |
| | | | | CN | 102548617 | B | 07 November 2017 |
| | | | | JP | 2017153478 | A | 07 September 2017 |
| | | | | BR | PI1014858 | A2 | 28 March 2017 |
| | | | | JP | 2015157856 | A | 03 September 2015 |
| | | | | EP | 2414043 | A1 | 08 February 2012 |
| | | | | US | 8338377 | B2 | 25 December 2012 |
| | | | | AU | 2010232693 | B2 | 21 April 2016 |
| | | | | KR | 101805201 | B1 | 05 December 2017 |
| | | | | EP | 3058986 | A1 | 24 August 2016 |
| | | | | US | 2018305438 | A1 | 25 October 2018 |
| | | | | AU | 2020204120 | A1 | 09 July 2020 |
| | | | | US | 2013101603 | A1 | 25 April 2013 |
| | | | | JP | 2012522507 | A | 27 September 2012 |
| | | | | AU | 2018201132 | A1 | 08 March 2018 |
| | | | | KR | 20120034604 | A | 12 April 2012 |
| | | | | EP | 2414043 | A4 | 24 October 2012 |
| | | | | ES | 2575695 | T3 | 30 June 2016 |
| CN | 102573921 | A | 11 July 2012 | WO | 2011056325 | A2 | 12 May 2011 |
| | | | | AU | 2010315805 | A1 | 15 March 2012 |
| | | | | JP | 5792175 | B2 | 07 October 2015 |
| | | | | EP | 2482855 | A2 | 08 August 2012 |
| | | | | CA | 2773353 | C | 27 February 2018 |
| | | | | IL | 218869 | A | 29 December 2016 |
| | | | | MX | 2012003775 | A | 12 June 2012 |
| | | | | AU | 2010315805 | B2 | 17 September 2015 |
| | | | | KR | 20120090961 | A | 17 August 2012 |
| | | | | KR | 101828613 | B1 | 12 February 2018 |
| | | | | JP | 2013505966 | A | 21 February 2013 |
| | | | | EP | 2482855 | B1 | 27 December 2017 |
| | | | | IL | 218869 | D0 | 28 June 2012 |
| | | | | CN | 102573921 | B | 25 November 2015 |

Form PCT/ISA/210 (patent family annex) (January 2015)

**INTERNATIONAL SEARCH REPORT**
Information on patent family members

| International application No. |
| --- |
| **PCT/CN2021/088266** |

| Patent document cited in search report | | | Publication date (day/month/year) | Patent family member(s) | | | Publication date (day/month/year) |
| --- | --- | --- | --- | --- | --- | --- | --- |
| | | | | CA | 2773353 | A1 | 12 May 2011 |
| | | | | MX | 341690 | B | 29 August 2016 |
| | | | | WO | 2011056325 | A3 | 29 September 2011 |
| CN | 102711789 | A | 03 October 2012 | HU | E030960 | T2 | 28 June 2017 |
| | | | | EP | 2459208 | A4 | 04 June 2014 |
| | | | | SM | P201700062 | B | 08 March 2017 |
| | | | | SI | EP2459208 | T1 | 26 April 2017 |
| | | | | US | 2012178688 | A1 | 12 July 2012 |
| | | | | CY | 1118388 | T1 | 28 June 2017 |
| | | | | EP | 3192520 | A1 | 19 July 2017 |
| | | | | HK | 1167108 | A1 | 23 November 2012 |
| | | | | PL | 3192520 | T3 | 30 August 2019 |
| | | | | EP | 3539555 | A1 | 18 September 2019 |
| | | | | JP | 5764613 | B2 | 19 August 2015 |
| | | | | EP | 2459208 | A2 | 06 June 2012 |
| | | | | LU | C00008 | I1 | 16 March 2017 |
| | | | | US | 2017114095 | A1 | 27 April 2017 |
| | | | | LT | PA2017007 | I1 | 27 March 2017 |
| | | | | SG | 178143 | A1 | 29 March 2012 |
| | | | | IL | 217749 | A | 29 September 2016 |
| | | | | CA | 2769525 | C | 21 February 2017 |
| | | | | NZ | 597922 | A | 30 January 2015 |
| | | | | LU | C00008 | I2 | 30 May 2017 |
| | | | | KR | 20120104513 | A | 21 September 2012 |
| | | | | SM | T201700014 | B | 08 March 2017 |
| | | | | RU | 2012107428 | A | 10 September 2013 |
| | | | | CN | 102711789 | B | 08 June 2018 |
| | | | | SM | P201700074 | B | 08 March 2017 |
| | | | | PT | 2459208 | T | 03 January 2017 |
| | | | | AU | 2010278897 | A1 | 23 February 2012 |
| | | | | DK | 3192520 | T3 | 27 May 2019 |
| | | | | JP | 2013500990 | A | 10 January 2013 |
| | | | | BR | 112012002143 | B1 | 08 December 2020 |
| | | | | LT | 2459208 | T | 27 February 2017 |
| | | | | LT | 3192520 | T | 25 June 2019 |
| | | | | CN | 107674114 | A | 09 February 2018 |
| | | | | ES | 2729051 | T3 | 30 October 2019 |
| | | | | US | 2013150297 | A1 | 13 June 2013 |
| | | | | ES | 2607954 | T3 | 04 April 2017 |
| | | | | US | 2016159860 | A1 | 09 June 2016 |
| | | | | US | 2015175664 | A1 | 25 June 2015 |
| | | | | US | 9701712 | B2 | 11 July 2017 |
| | | | | RU | 2557654 | C3 | 05 July 2017 |
| | | | | JP | 5270799 | B2 | 21 August 2013 |
| | | | | US | 2020399309 | A1 | 24 December 2020 |
| | | | | US | 9278995 | B2 | 08 March 2016 |
| | | | | SI | 2459208 | T1 | 26 April 2017 |
| | | | | JP | 2014094967 | A | 22 May 2014 |
| | | | | AU | 2010278897 | B2 | 26 February 2015 |
| | | | | MX | 2012001213 | A | 12 June 2012 |

Form PCT/ISA/210 (patent family annex) (January 2015)

INTERNATIONAL SEARCH REPORT
Information on patent family members

International application No.

**PCT/CN2021/088266**

| Patent document cited in search report | | | Publication date (day/month/year) | Patent family member(s) | | | Publication date (day/month/year) |
|---|---|---|---|---|---|---|---|
| | | | | US | 2011028394 | A1 | 03 February 2011 |
| | | | | EP | 3192520 | B1 | 06 March 2019 |
| CN | 110054662 | A | 26 July 2019 | CN | 110054662 | B | 02 March 2021 |
| WO | 2018152542 | A1 | 23 August 2018 | US | 2020202980 | A1 | 25 June 2020 |
| WO | 2005100571 | A1 | 27 October 2005 | JP | 2008505614 | A | 28 February 2008 |
| | | | | US | 2008064630 | A1 | 13 March 2008 |
| | | | | EP | 1740701 | A1 | 10 January 2007 |
| US | 2004126357 | A1 | 01 July 2004 | US | 2018237487 | A1 | 23 August 2018 |
| | | | | US | 2004170960 | A1 | 02 September 2004 |
| | | | | US | 9951113 | B2 | 24 April 2018 |
| | | | | US | 2010292453 | A1 | 18 November 2010 |
| | | | | US | 2004241137 | A1 | 02 December 2004 |
| | | | | US | 2005064391 | A1 | 24 March 2005 |
| | | | | US | 2004142889 | A1 | 22 July 2004 |
| | | | | US | 2004122217 | A1 | 24 June 2004 |
| | | | | US | 2004126793 | A1 | 01 July 2004 |
| | | | | US | 2004151728 | A1 | 05 August 2004 |
| | | | | US | 8940307 | B2 | 27 January 2015 |
| | | | | US | 2004180389 | A1 | 16 September 2004 |
| | | | | US | 2004091503 | A1 | 13 May 2004 |
| | | | | US | 2017088595 | A1 | 30 March 2017 |
| | | | | US | 2015297715 | A1 | 22 October 2015 |
| | | | | US | 9597394 | B2 | 21 March 2017 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010946404